# EUROPEAN PATENT APPLICATION

(11) **EP 4 585 227 A1**
(43) Date of publication of application: **16.07.2025**
(21) Application number: 23862373.0
(22) Date of filing: 05.09.2023
(51) Int. Cl.: A61K 45/06, A61K 31/506, A61K 31/444, A61K 39/395, A61P 35/00, A61K 39/00

(54) **PHARMACEUTICAL COMBINATION OF FAK INHIBITOR AND EGFR-TKI, AND USE**

(30) Priority: 05.09.2022 CN 202211077426; 23.09.2022 CN 202211166052; 08.08.2023 CN 202310998195
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); GAO, Jiaming, Nanjing, Jiangsu 210061 (CN); ZHANG, Ping, Nanjing, Jiangsu 210061 (CN); PANG, Ran, Nanjing, Jiangsu 210061 (CN); WANG, Zaiqi, Nanjing, Jiangsu 210061 (CN)
(74) Representative: D Young & Co LLP
(86) International application number: PCT/CN2023/116969
(87) International publication number: WO 2024/051679

(57) **Abstract**

An FAK inhibitor, an epidermal growth factor receptor tyrosine kinase inhibitor, and an immune checkpoint inhibitor are combined to treat tumors, and an FAK inhibitor and an epidermal growth factor receptor tyrosine kinase inhibitor are combined to treat tumors.

## Description

This application claims the priority of Chinese Patent Application No. 202211077426.8 filed on September 5, 2022, Chinese Patent Application No. 202211166052.7 filed on September 23, 2022, and Chinese Patent Application No. 202310998195.2 filed on August 8, 2023. The disclosures of the above Chinese patent applications are hereby cited in their entirety as part of this application.

### TECHNICAL FIELD

The present disclosure belongs to the field of medicinal chemistry. Specifically, the present disclosure relates to the treatment of a tumor using a focal adhesion kinase (FAK) inhibitor in combination with other drugs.

### BACKGROUND

Tumor is the second biggest killer threatening people's health. Immunogenic cell death (ICD) is an additive effect based on the programmed cell death. An intracellular stress signal may be activated when cancer cells are exposed to chemotherapy or targeted medicaments. This kind of stress signal includes endoplasmic reticulum stress (ER stress) and oxidative stress. Under the action of the stress signal, cells will try to repair the stress first. The cells will start the programmed death process, if the damage caused by the stress exceeds the repair ability of the cells. This process is often accompanied by the release of a class of molecules called damage associated molecular patterns (DAMPs), which include calreticulin, annexin A1, high mobility group box 1 (HMGB1), etc. These DAMPs will be specifically recognized by pattern recognition receptors on antigen-presenting cells (APCs) in the body, which will induce the maturation, differentiation and activation of APCs, and then gradually present them to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce an anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

EGFR is the abbreviation of epidermal growth factor receptor. The EGFR gene is responsible for encoding and making a receptor protein called epidermal growth factor receptor. The EGFR protein is a transmembrane protein that is divided into three parts: one end of the protein is located outside the cell, one part is located in the cell membrane, and the other end is located inside the cell. This allows the EGFR to bind to other proteins outside the cell (called ligands), helping the cell receive signals and respond to the stimulation. The binding of a receptor to a ligand is like a key to a lock, so they both have specific binding "partners". When an EGFR binds to a ligand, it attaches to another EGFR located nearby and forms a complex (a dimer), thereby entering an activated state and activating the signaling pathway in the cell. EGFR mutations mainly occur in exons 18 to 21, among which deletion mutations in exon 19 and L858R point mutations in exon 21 are the most common mutation types, accounting for 90% of all the mutation types. When a pathogenic gene mutation occurs in EGFR, the EGFR protein will be in a continuously activated state, which causes the cell to continuously receive proliferation and survival signals, resulting in excessive cell growth and survival (inability to undergo normal apoptosis), leading to the tumor formation.

Currently, the targeted drugs for EGFR mutations on the market include: the first-generation targeted drugs Icotinib, Gefitinib, and Erlotinib targeting mutations in exon 19 and exon 21; the second-generation targeted drugs Afatinib targeting mutations in exon 8 and exon 20; and the third-generation targeted drugs Osimertinib (also referred to as AZD9291 herein), Almonertinib, and Alflutinib targeting T790M mutations. Targeted drugs for ALK mutations include: the first-generation targeted drug Crizotinib, the second-generation targeted drugs Ceritinib, Alectinib, Brigatinib, and the third-generation targeted drugs Lorlatinib, etc. However, drug resistance to these targeted drugs often appears about 1 year after the medication. Overcoming the resistance to targeted drugs, or delaying the onset of drug resistance and improving the likelihood of cure are the main goals of drug development.

Therefore, there is still a need to find a way to improve the efficacy of single targeted drugs, increase the response rate, create conditions for immunotherapy through combination therapy, and further overcome drug resistance to provide the possibility of tumor cure.

### SUMMARY

One aspect of the present disclosure provides use of an FAK inhibitor, an epidermal growth factor receptor tyrosine kinase inhibitor, and an immune checkpoint inhibitor in the manufacture of a medicament for treating a tumor in a subject.

Another aspect of the present disclosure provides a pharmaceutical combination product of an FAK inhibitor, an epidermal growth factor receptor tyrosine kinase inhibitor, and an immune checkpoint inhibitor, for use in treating a tumor in a subject.

Another aspect of the present disclosure provides a method of treating a tumor, comprising administering a therapeutically effective amount of an FAK inhibitor, an epidermal growth factor receptor tyrosine kinase inhibitor, and an immune checkpoint inhibitor to a subject in need.

Another aspect of the present disclosure provides a kit or a pharmaceutically acceptable composition, comprising: (a) an FAK inhibitor; (b) an epidermal growth factor receptor tyrosine kinase inhibitor; and (c) an immune checkpoint inhibitor.

Another aspect of the present disclosure provides use of an FAK inhibitor and an epidermal growth factor receptor tyrosine kinase inhibitor in the manufacture of a medicament for treating a tumor, wherein the FAK inhibitor is used to enhance the immunogenic cell death induced by the epidermal growth factor receptor tyrosine kinase inhibitor.

Another aspect of the present disclosure provides an FAK inhibitor, for use in enhancing the immunogenic cell death induced by an epidermal growth factor receptor tyrosine kinase inhibitor in the treatment of a tumor.

Another aspect of the present disclosure provides a method of treating a tumor, comprising administering a therapeutically effective amount of an FAK inhibitor and an epidermal growth factor receptor tyrosine kinase inhibitor to a subject in need, wherein the FAK inhibitor is used to enhance the immunogenic cell death induced by the epidermal growth factor receptor tyrosine kinase inhibitor.

Another aspect of the present disclosure provides use of an FAK inhibitor, an epidermal growth factor receptor tyrosine kinase inhibitor, and an immune checkpoint inhibitor in the manufacture of a medicament for the combined treatment of a tumor.

Another aspect of the present disclosure provides use of an FAK inhibitor in the manufacture of a combined medicament with an epidermal growth factor receptor tyrosine kinase inhibitor and an immune checkpoint inhibitor for treating a tumor.

Another aspect of the present disclosure provides use of an epidermal growth factor receptor tyrosine kinase inhibitor in the manufacture of a combined medicament with an FAK inhibitor and an immune checkpoint inhibitor for treating a tumor.

Another aspect of the present disclosure provides use of an immune checkpoint inhibitor in the manufacture of a combined medicament with an FAK inhibitor and an epidermal growth factor receptor tyrosine kinase inhibitor for treating a tumor.

Another aspect of the present disclosure provides use of an FAK inhibitor in the manufacture of a medicament for the combined treatment of a tumor with an epidermal growth factor receptor tyrosine kinase inhibitor and an immune checkpoint inhibitor.

Another aspect of the present disclosure provides use of an epidermal growth factor receptor tyrosine kinase inhibitor in the manufacture of a medicament for the combined treatment of a tumor with an FAK inhibitor and an immune checkpoint inhibitor.

Another aspect of the present disclosure provides use of an immune checkpoint inhibitor in the manufacture of a medicament for the combined treatment of a tumor with an FAK inhibitor and an epidermal growth factor receptor tyrosine kinase inhibitor.

Another aspect of the present disclosure provides a kit comprising: an FAK inhibitor; and instructions, which indicate that the FAK inhibitor can be used for the combined treatment of a tumor with an epidermal growth factor receptor tyrosine kinase inhibitor and an immune checkpoint inhibitor.

Another aspect of the present disclosure provides a kit comprising: an epidermal growth factor receptor tyrosine kinase inhibitor; and instructions, which indicate that the epidermal growth factor receptor tyrosine kinase inhibitor can be used for the combined treatment of a tumor with an FAK inhibitor and an immune checkpoint inhibitor.

Another aspect of the present disclosure provides a kit comprising: an immune checkpoint inhibitor; and instructions, which indicate that the immune checkpoint inhibitor can be used for the combined treatment of a tumor with an FAK inhibitor and an epidermal growth factor receptor tyrosine kinase inhibitor.

Another aspect of the present disclosure provides a method of treating a tumor, comprising administering a therapeutically effective amount of an FAK inhibitor and an epidermal growth factor receptor tyrosine kinase inhibitor to a subject in need.

Another aspect of the present disclosure provides a pharmaceutical combination product of an FAK inhibitor and an epidermal growth factor receptor tyrosine kinase inhibitor, which is used for treating a tumor in a subject in need.

Another aspect of the present disclosure provides use of an FAK inhibitor and an epidermal growth factor receptor tyrosine kinase inhibitor in the manufacture of a combined medicament for treating a tumor.

Another aspect of the present disclosure provides use of an FAK inhibitor in the manufacture of a combined medicament with an epidermal growth factor receptor tyrosine kinase inhibitor for treating a tumor.

Another aspect of the present disclosure provides use of an epidermal growth factor receptor tyrosine kinase inhibitor in the manufacture of a combined medicament with an FAK inhibitor for treating a tumor.

Another aspect of the present disclosure provides use of an FAK inhibitor and an epidermal growth factor receptor tyrosine kinase inhibitor in the manufacture of a medicament for the combined treatment of a tumor.

Another aspect of the present disclosure provides use of an FAK inhibitor in the manufacture of a medicament for the combined treatment of a tumor with an epidermal growth factor receptor tyrosine kinase inhibitor.

Another aspect of the present disclosure provides use of an epidermal growth factor receptor tyrosine kinase inhibitor in the manufacture of a medicament for the combined treatment of a tumor with an FAK inhibitor.

Another aspect of the present disclosure provides a kit comprising: an FAK inhibitor; and instructions, which indicate that the FAK inhibitor can be used for the combined treatment of a tumor with an epidermal growth factor receptor tyrosine kinase inhibitor.

Another aspect of the present disclosure provides a kit comprising: an epidermal growth factor receptor tyrosine kinase inhibitor; and instructions, which indicate that the epidermal growth factor receptor tyrosine kinase inhibitor can be used for the combined treatment of a tumor with an FAK inhibitor.

Optionally, the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows:

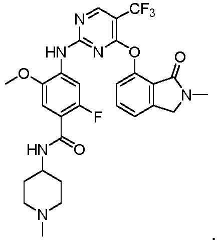

The Defactinib is also known as difatini, and the CAS number is 1345713-71-4; the CAS number of GSK2256098 is 1224887-10-8. The CAS number of PF-00562271 is 717907-75-0; the CAS number of VS-4718 is 1061353-68-1; the APG-2449 is developed by Ascentage Pharma; and the CAS number of AMP945 is 1393653-34-3.

Optionally, the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.

The CAS number of Gefitinib is 184475-35-2; the CAS number of Erlotinib is 183321-74-6; the CAS number of Icotinib is 610798-31-7; the CAS number of Afatinib is 850140-72-6; the CAS number of Crizotinib is 877399-52-5; the CAS number of Osimertinib (AZD9291) is 1421373-65-0; the CAS number of Almonertinib is 1899921-05-1; the CAS number of Alflutinib (also known as Furmonertinib) is 1869057-83-9; the CAS number of EAI045 is 1942114-09-1; the CAS number of JBJ-04-125-02 is 2060610-53-7; the CAS number of BLU-945 is 2660250-10-0; BLU-701 is jointly developed by Blueprint Medicines Corp and Zai Lab; the CAS number of TQB3804 is 2267329-76-8; BBT-176 is developed by Bridge Biotherapeutics, Inc.; ES-072 is developed by Bosheng Pharmaceutical Co Ltd.; BPI-361175 is developed by Betta Pharmaceuticals; and CH7233163 is developed by Chugai Pharmaceutical Co., Ltd.

Optionally, the epidermal growth factor receptor tyrosine kinase inhibitor is Osimertinib, Almonertinib, Alflutinib, or a pharmaceutically acceptable salt thereof.

Optionally, the epidermal growth factor receptor tyrosine kinase inhibitor is Osimertinib or a pharmaceutically acceptable salt thereof.

Optionally, the epidermal growth factor receptor tyrosine kinase inhibitor is Almonertinib or a pharmaceutically acceptable salt thereof.

Optionally, the epidermal growth factor receptor tyrosine kinase inhibitor is Alflutinib or a pharmaceutically acceptable salt thereof.

Optionally, the immune checkpoint inhibitor is an anti-PD-1/PD-L1 antibody, a PD-1/PD-L1 small molecule inhibitor, or a TIGIT inhibitor.

Optionally, the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.

Optionally, the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.

Optionally, the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

Optionally, the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody.

Optionally, the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody.

Optionally, the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody.

Optionally, the FAK inhibitor, the epidermal growth factor receptor tyrosine kinase inhibitor and the immune checkpoint inhibitor are administered to the subject simultaneously or sequentially.

Optionally, the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML).

Optionally, the tumor is alternatively breast cancer, ovarian cancer, colon cancer (including colorectal cancer), lung cancer (including small cell lung cancer and non-small cell lung cancer), melanoma or pancreatic cancer.

Optionally, the tumor is lung cancer (including small cell lung cancer and non-small cell lung cancer) or colon cancer (including colorectal cancer).

Optionally, the tumor is non-small cell lung cancer or colon cancer (including colorectal cancer).

### BRIEF DESCRIPTION OF DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.
Fig. 1 shows a white light microscope photograph of lung cancer KPL cells after being incubated with a medicament for 48 hours in example 1.
Fig. 2a shows the percentage of CRT positive KPL cells after the lung cancer KPL cells were incubated with the medicament for 48 hours in example 1; and Fig. 2b shows the percentage of Annexin V positive KPL cells after the lung cancer KPL cells were incubated with the medicament for 48 hours in example 1.
Fig. 3 shows a white light microscope photograph of lung cancer KPL cells after being incubated with a medicament for 48 hours in example 2.
Fig. 4a shows the percentage of CRT positive KPL cells after the lung cancer KPL cells were incubated with the medicament for 48 hours in example 2; and Fig. 4b shows the percentage of Annexin V positive KPL cells after the lung cancer KPL cells were incubated with the medicament for 48 hours in example 2.
Fig. 5 shows a white light microscope photograph of lung cancer KPL cells after being incubated with a medicament for 48 hours in example 3.
Fig. 6a shows the percentage of CRT positive KPL cells after the lung cancer KPL cells were incubated with the medicament for 48 hours in example 3; and Fig. 6b shows the percentage of Annexin V positive KPL cells after the lung cancer KPL cells were incubated with the medicament for 48 hours in example 3.
Fig. 7 shows the early and late apoptosis status of the cells after FAK silencing combined with the treatment of 0.3 nM AZD9291 for 48 hours.
Fig. 8 shows the enhanced release and exposure of Calreticulin after FAK silencing combined with the treatment of 0.3 nM AZD9291 for 48 hours.
Fig. 9 shows the synergistic killing effect of AZD9291 at different dosages combined with 3 µM IN10018 and 5 µM IN10018 for 48 hours, respectively, on non-small cell lung cancer cells HCC827.
Fig. 10 shows the detection of cell apoptosis after 48 hours of combined treatment of 0.3 nM AZD9291 and 3 µM IN10018.
Fig. 11 shows that the combination of AZD9291 and IN10018 significantly upregulated the endoplasmic reticulum stress of HCC827.
Fig. 12 shows that the combination of AZD9291 and IN10018 enhanced the Calreticulin release and exposure.
Fig. 13 shows the changes in tumor volume after administration of different assay substances in the subcutaneous allograft tumor model of colon cancer CT26 cells in BALB/c mice.
Fig. 14 shows the changes in body weight of mice after administration of different assay substances in the subcutaneous allograft tumor model of colon cancer CT26 cells in BALB/c mice.
Fig. 15 shows the changes in tumor volume after administration of different assay substances in the subcutaneous allograft tumor model of colon cancer MC38 cells in C57BL/6 mice.
Fig. 16 shows the changes in body weight of mice after administration of different assay substances in the subcutaneous allograft tumor model of colon cancer MC38 cells in C57BL/6 mice.
Fig. 17a shows the percentage of CRT positive 4T1 cells after the breast cancer 4T1 cells were incubated with the medicament for 48 hours in example 8; and Fig. 17b shows the percentage of Annexin V positive 4T1 cells after the breast cancer 4T1 cells were incubated with the medicament for 48 hours in example 8.
Fig. 18a shows the percentage of CRT positive 4T1 cells after the breast cancer 4T1 cells were incubated with the medicament for 48 hours in example 9; and Fig. 18b shows the percentage of Annexin V positive 4T1 cells after the breast cancer 4T1 cells were incubated with the medicament for 48 hours in example 9.
Fig. 19a shows the percentage of CRT positive 4T1 cells after the breast cancer 4T1 cells were incubated with the medicament for 48 hours in example 10; and Fig. 19b shows the percentage of Annexin V positive 4T1 cells after the breast cancer 4T1 cells were incubated with the medicament for 48 hours in example 10.

### DETAILED DESCRIPTION

In order to make the purpose, technical solutions and advantages of the examples of the present disclosure clearer, the technical solutions of the examples of the present disclosure will be described clearly and completely with the attached figures of the examples of the present disclosure. Obviously, the described examples are parts of examples of the present disclosure, not the whole examples. Based on the described examples of the present disclosure, all other examples obtained by those of ordinary skill in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes the further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing the article and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the specification and claims should be understood as being modified in all instances by the term "about". It should also be understood that any numerical range recited in this application is intended to include all subranges within the range and any combination of endpoints of the range or subranges.

"Including", "containing", "comprising" or the like used in this disclosure means that the elements appearing before the word cover the elements listed after the word and their equivalents, but do not exclude elements that are not recorded. The term "containing" or "including (comprising)" used herein can be open, semi-closed or closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, especially humans. In some embodiments, the FAK inhibitor is IN10018, Defactinib, GSK2256098, PF-00562271, VS-4718, APG-2449, AMP945, AMP886, or a pharmaceutically acceptable salt thereof, wherein structure of the IN10018 is as follows: 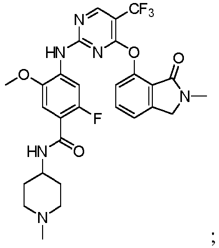 the Defactinib is also known as difatini, and the CAS number is 1345713-71-4; the CAS number of GSK2256098 is 1224887-10-8. The CAS number of PF-00562271 is 717907-75-0; the CAS number of VS-4718 is 1061353-68-1; the APG-2449 is developed by Ascentage Pharma; and the CAS number of AMP945 is 1393653-34-3.

In some embodiments, the FAK inhibitor is alternatively IN10018, AMP945, Defactinib, or a pharmaceutically acceptable salt thereof, and in some alternative embodiments, the FAK inhibitor is IN10018 or a pharmaceutically acceptable salt thereof, alternatively, the FAK inhibitor is a tartrate of IN10018.

As used herein, the term "epidermal growth factor receptor tyrosine kinase inhibitor (EGFR-TKI)" is an agent that selectively and effectively inhibits epidermal growth factor receptor tyrosine kinase. Examples of the epidermal growth factor receptor tyrosine kinase inhibitor include, but are not limited to, Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, and pharmaceutically acceptable salts thereof. In some embodiments, the epidermal growth factor receptor tyrosine kinase inhibitor is Osimertinib, Almonertinib, Alflutinib, or a pharmaceutically acceptable salt thereof.

The CAS number of Gefitinib is 184475-35-2; the CAS number of Erlotinib is 183321-74-6; the CAS number of Icotinib is 610798-31-7; the CAS number of Afatinib is 850140-72-6; the CAS number of Dacomitinib is 1110813-31-4; the CAS number of Crizotinib is 877399-52-5; the CAS number of Osimertinib (AZD9291) is 1421373-65-0; the CAS number of Almonertinib is 1899921-05-1; the CAS number of Alflutinib (also known as Furmonertinib) is 1869057-83-9; the CAS number of EAI045 is 1942114-09-1; the CAS number of JBJ-04-125-02 is 2060610-53-7; the CAS number of BLU-945 is 2660250-10-0; BLU-701 is jointly developed by Blueprint Medicines Corp and Zai Lab; the CAS number of TQB3804 is 2267329-76-8; BBT-176 is developed by Bridge Biotherapeutics, Inc.; ES-072 is developed by Bosheng Pharmaceutical Co Ltd.; BPI-361175 is developed by Betta Pharmaceuticals; and CH7233163 is developed by Chugai Pharmaceutical Co., Ltd.

As used herein, the term "immune checkpoint inhibitor" refers to a medicament that can improve the immune system activity by regulating immune checkpoint pathways (such as PD-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor"), or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is an anti-PD-1/PD-L1 antibody, including, but not limited to, Pembrolizumab (kerida/Keytruda), Tislelizumab (Baizean), Nivolumab, Toripalimab (Tuoyi), Durvalumab (Imfinzi), Avelumab (Bavencio), Atezolizumab (MPDL 3280A/Tecentriq), BMS-936559 (fully humanized IgG4 monoclonal antibody against PD-L1), GS-4224, AN-4005, or MX-10181. In some alternative embodiments, the PD-1 inhibitor is Toripalimab. In some embodiments, the PD-1 inhibitor is used to treat a human subject. In some embodiments, the PD-1 is human PD-1. The PD-1/PD-L1 inhibitors also include PD-1/PD-L1 small molecule inhibitors, such as INCB-086550, Lazertinib, IMMH-010, CA-170, ABSK043, or RRx-001. TIGIT (also known as WUCAM, Vstm3, or VSIG9) is a receptor of the Ig superfamily and is a new immune checkpoint besides PD-1/PD-L1. For example, in the therapeutic methods, medicaments and uses of the present disclosure, the immune checkpoint inhibitor is a TIGIT inhibitor, including, but not limited to, Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, HB0036, or IBI-321. In some embodiments, the TIGIT inhibitor is used to treat a human subject. In order to avoid ambiguity, all antibodies in the present disclosure include bispecific antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combination therapy" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week, or 24 hours). The modes of administration (for example, oral administration, intravenous administration, intramuscular administration, or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously, or orally. Alternatively, the PD-1/PD-L1 inhibitor is administered by intravenous infusion.

In some embodiments, the TIGIT inhibitor is administered intravenously (e.g., as intravenous infusion), subcutaneously, or orally. Alternatively, the TIGIT inhibitor is administered by intravenous infusion.

The ability of immune checkpoint inhibitors to treat a cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, especially when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate, or influence the disease or symptoms of the disease. In some embodiments, the disease is a tumor or cancer.

As used herein, the term "tumor" refers to the abnormal pathological changes formed by the abnormal proliferation of clonal cells caused by the loss of normal regulation of the growth of cells of local tissues at the gene level under the action of various tumorigenic factors. The tumors include, but are not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma, and hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); in some embodiments, the tumor is alternatively breast cancer, ovarian cancer, colon cancer (including colorectal cancer), lung cancer (including small cell lung cancer and non-small cell lung cancer), melanoma or pancreatic cancer; in some embodiments, the tumor is lung cancer (including small cell lung cancer and non-small cell lung cancer) or colon cancer (including colorectal cancer); in some embodiments, the tumor is non-small cell lung cancer or colon cancer (including colorectal cancer).

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts formed with organic acids, such as formate, acetate, malate, maleate, fumarate, tartrate, succinate, citrate, lactate, methane sulfonate, p-toluenesulfonate, 2-hydroxyethyl sulfonate, benzoate, salicylate, stearate and salts formed with alkanedicarboxylic acids of formula HOOC-(CH₂)ₙ-COOH (wherein n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the preparation, and/or compatible with the subject to be treated with it. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of an FAK inhibitor, an epidermal growth factor receptor tyrosine kinase inhibitor and an immune checkpoint inhibitor; and (ii) instructions indicating that the FAK inhibitor, the epidermal growth factor receptor tyrosine kinase inhibitor and the immune checkpoint inhibitor can be used to treat a tumor in a subject. In one embodiment, the kit comprises (i) an FAK inhibitor; and (ii) instructions indicating that the FAK inhibitor, an epidermal growth factor receptor tyrosine kinase inhibitor and an immune checkpoint inhibitor can be used to treat a tumor in a subject. In one embodiment, the kit comprises (i) an epidermal growth factor receptor tyrosine kinase inhibitor; and (ii) instructions indicating that an FAK inhibitor, the epidermal growth factor receptor tyrosine kinase inhibitor and an immune checkpoint inhibitor can be used to treat a tumor in a subject. In one embodiment, the kit comprises (i) an immune checkpoint inhibitor; and (ii) instructions indicating that an FAK inhibitor, an epidermal growth factor receptor tyrosine kinase inhibitor and the immune checkpoint inhibitor can be used to treat a tumor in a subject. In one embodiment, the kit comprises (i) an FAK inhibitor, an epidermal growth factor receptor tyrosine kinase inhibitor and an immune checkpoint inhibitor; and (ii) instructions indicating that the FAK inhibitor, the epidermal growth factor receptor tyrosine kinase inhibitor and the immune checkpoint inhibitor can be used to treat a tumor in a subject. In one embodiment, the kit comprises (i) an FAK inhibitor; and (ii) instructions indicating that the FAK inhibitor and an epidermal growth factor receptor tyrosine kinase inhibitor can be used to treat a tumor in a subject. In one embodiment, the kit comprises (i) an epidermal growth factor receptor tyrosine kinase inhibitor; and (ii) instructions indicating that the epidermal growth factor receptor tyrosine kinase inhibitor and an FAK inhibitor can be used to treat a tumor in a subject.

The compounds of a kit can be contained in separate containers. Optionally, two or more compounds are contained in the same container. For example, the kit may include a first container, a second container, a third container, and a package insert, wherein the first container includes at least one dose of a medicament including an FAK inhibitor, the second container includes at least one dose of an epidermal growth factor receptor tyrosine kinase inhibitor, and the third container includes at least one dose of a medicament of immune checkpoint inhibitor, and the package insert includes instructions of treating a tumor in a subject with the medicaments. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the epidermal growth factor receptor tyrosine kinase inhibitor or the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the epidermal growth factor receptor tyrosine kinase inhibitor, or the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups, or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with the required various other components mentioned above into a suitable solvent, and then filtering and sterilizing the mixture. For the sterile powder used to prepare the sterile injection solution, the alternative preparation methods can be vacuum drying and freeze-drying techniques, which can produce powder of active ingredient plus any other required ingredients existing after previous sterile filtration.

The amount of the FAK inhibitor, the epidermal growth factor receptor tyrosine kinase inhibitor or the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of approximately 0.1 to approximately 50mg/kg body weight per day.

The FAK inhibitor is administered in a dosage range of 5mg/day to 300 mg/day in adults. In a specific embodiment, IN10018 or a pharmaceutically acceptable salt thereof is administered at a dosage of 5 mg/day to 100 mg/day in adults. In a specific embodiment, IN10018 or a pharmaceutically acceptable salt thereof is administered at a dosage of 25 mg/day to 100 mg/day in adults, and the dosage is calculated based on the free base.

The epidermal growth factor receptor tyrosine kinase inhibitor is administered in a dosage range of 2 to 250 mg per day in adults. In a specific embodiment, Osimertinib or a pharmaceutically acceptable salt thereof is administered at a dosage of 2 to 250 mg, for example, 80 mg, per day in adults, and the dosage is calculated based on Osimertinib; Almonertinib or a pharmaceutically acceptable salt thereof is administered at a dosage of 2 to 250 mg, for example, 110 mg, per day in adults, and the dosage is calculated based on Almonertinib; Alflutinib or a pharmaceutically acceptable salt thereof is administered at a dosage of 2 to 250 mg, for example, 80 mg, per day in adults, and the dosage is calculated based on Alflutinib.

The immune checkpoint inhibitor is administered each time at a dosage of 2 to 10mg/kg or 50 to 1200mg in adults, and administered every 2 to 3 weeks. In a specific embodiment, the immune checkpoint inhibitor is administered each time at a dosage of 3 to 10mg/kg or 100 to 1200mg in adults, and administered every 2 to 3 weeks.

Technical and scientific terms without specific definition used herein have the meanings commonly understood by those skilled in the art to which this disclosure belongs.

In some embodiments, the present disclosure also discloses:
1. An FAK inhibitor, the epidermal growth factor receptor tyrosine kinase inhibitor and an immune checkpoint inhibitor, for use in a method of treating a tumor in a subject.
2. The FAK inhibitor, epidermal growth factor receptor tyrosine kinase inhibitor and immune checkpoint inhibitor according to embodiment 1, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 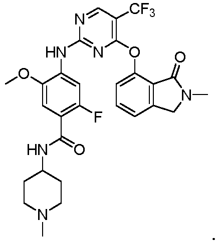
3. The FAK inhibitor, epidermal growth factor receptor tyrosine kinase inhibitor and immune checkpoint inhibitor according to embodiment 1 or 2, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
4. The FAK inhibitor, epidermal growth factor receptor tyrosine kinase inhibitor and immune checkpoint inhibitor according to any one of embodiments 1 to 3, wherein the epidermal growth factor receptor tyrosine kinase inhibitor is Osimertinib, Almonertinib, Alflutinib, or a pharmaceutically acceptable salt thereof.
5. The FAK inhibitor, epidermal growth factor receptor tyrosine kinase inhibitor and immune checkpoint inhibitor according to any one of embodiments 1 to 4, wherein the epidermal growth factor receptor tyrosine kinase inhibitor is Osimertinib or a pharmaceutically acceptable salt thereof.
6. The FAK inhibitor, epidermal growth factor receptor tyrosine kinase inhibitor and immune checkpoint inhibitor according to any one of embodiments 1 to 4, wherein the epidermal growth factor receptor tyrosine kinase inhibitor is Almonertinib or a pharmaceutically acceptable salt thereof.
7. The FAK inhibitor, epidermal growth factor receptor tyrosine kinase inhibitor and immune checkpoint inhibitor according to any one of embodiments 1 to 4, wherein the epidermal growth factor receptor tyrosine kinase inhibitor is Alflutinib or a pharmaceutically acceptable salt thereof.
8. The FAK inhibitor, epidermal growth factor receptor tyrosine kinase inhibitor and immune checkpoint inhibitor according to any one of embodiments 1 to 7, wherein the immune checkpoint inhibitor is an anti-PD-1/PD-L1 antibody, a PD-1/PD-L1 small molecule inhibitor, or a TIGIT inhibitor.
9. The FAK inhibitor, epidermal growth factor receptor tyrosine kinase inhibitor and immune checkpoint inhibitor according to any one of embodiments 1 to 8, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
10. The use, pharmaceutical combination product or method according to any one of embodiments 1 to 8, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
11. The FAK inhibitor, epidermal growth factor receptor tyrosine kinase inhibitor and immune checkpoint inhibitor according to any one of embodiments 1 to 8, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
12. The FAK inhibitor, epidermal growth factor receptor tyrosine kinase inhibitor and immune checkpoint inhibitor according to any one of embodiments 1 to 4, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
13. The FAK inhibitor, epidermal growth factor receptor tyrosine kinase inhibitor and immune checkpoint inhibitor according to any one of embodiments 1 to 4, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
14. The FAK inhibitor, epidermal growth factor receptor tyrosine kinase inhibitor and immune checkpoint inhibitor according to any one of embodiments 1 to 4, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
15. The FAK inhibitor, epidermal growth factor receptor tyrosine kinase inhibitor and immune checkpoint inhibitor according to any one of embodiments 1 to 14, wherein the FAK inhibitor, the epidermal growth factor receptor tyrosine kinase inhibitor, and the immune checkpoint inhibitor are administered to the subject simultaneously or sequentially.
16. The FAK inhibitor, epidermal growth factor receptor tyrosine kinase inhibitor and immune checkpoint inhibitor according to any one of embodiments 1 to 15, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
17. The FAK inhibitor, epidermal growth factor receptor tyrosine kinase inhibitor and immune checkpoint inhibitor according to any one of embodiments 1 to 16, wherein the tumor is lung cancer (including small cell lung cancer and non-small cell lung cancer) or colon cancer (including colorectal cancer), alternatively, the tumor is non-small cell lung cancer or colon cancer (including colorectal cancer).
18. A kit or a pharmaceutically acceptable composition, comprising:
   (a) an FAK inhibitor;
   (b) an epidermal growth factor receptor tyrosine kinase inhibitor; and
   (c) an immune checkpoint inhibitor.
19. The kit or composition according to embodiment 18, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 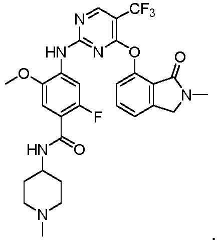
20. The kit or composition according to embodiment 18 or 19, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
21. The kit or composition according to any one of embodiments 18 to 20, wherein the epidermal growth factor receptor tyrosine kinase inhibitor is Osimertinib, Almonertinib, Alflutinib, or a pharmaceutically acceptable salt thereof.
22. The kit or composition according to any one of embodiments 18 to 21, wherein the epidermal growth factor receptor tyrosine kinase inhibitor is Osimertinib or a pharmaceutically acceptable salt thereof.
23. The kit or composition according to any one of embodiments 18 to 21, wherein the epidermal growth factor receptor tyrosine kinase inhibitor is Almonertinib or a pharmaceutically acceptable salt thereof.
24. The composition according to any one of embodiments 18 to 21, wherein the epidermal growth factor receptor tyrosine kinase inhibitor is Alflutinib or a pharmaceutically acceptable salt thereof.
25. The kit or composition according to any one of embodiments 18 to 24, wherein the immune checkpoint inhibitor is an anti-PD-1/PD-L1 antibody, a PD-1/PD-L1 small molecule inhibitor, or a TIGIT inhibitor.
26. The kit or composition according to any one of embodiments 18 to 25, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
27. The use, pharmaceutical combination product or method according to any one of embodiments 18 to 25, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
28. The kit or composition according to any one of embodiments 18 to 25, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
29. The kit or composition according to any one of embodiments 18 to 21, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
30. The kit or composition according to any one of embodiments 18 to 21, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
31. The kit or composition according to any one of embodiments 18 to 21, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
32. The kit or composition according to any one of embodiments 18 to 31, wherein the composition is for use as a medicament.
33. The kit or composition according to embodiment 32, wherein the medicament is used to treat a tumor, and the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
34. The kit or composition according to embodiment 33, wherein the tumor is lung cancer (including small cell lung cancer and non-small cell lung cancer) or colon cancer (including colorectal cancer), alternatively, the tumor is non-small cell lung cancer or colon cancer (including colorectal cancer).
35. A method of treating a tumor in a subject, wherein the method comprises administering a therapeutically effective amount of an FAK inhibitor, an epidermal growth factor receptor tyrosine kinase inhibitor, and an immune checkpoint inhibitor.
36. The method according to embodiment 35, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 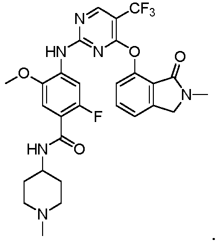
37. The method according to embodiment 35 or 36, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
38. The method according to any one of embodiments 35 to 37, wherein the epidermal growth factor receptor tyrosine kinase inhibitor is Osimertinib, Almonertinib, Alflutinib, or a pharmaceutically acceptable salt thereof.
39. The method according to any one of embodiments 35 to 38, wherein the epidermal growth factor receptor tyrosine kinase inhibitor is Osimertinib or a pharmaceutically acceptable salt thereof.
40. The method according to any one of embodiments 35 to 38, wherein the epidermal growth factor receptor tyrosine kinase inhibitor is Almonertinib or a pharmaceutically acceptable salt thereof.
41. The method according to any one of embodiments 35 to 38, wherein the epidermal growth factor receptor tyrosine kinase inhibitor is Alflutinib or a pharmaceutically acceptable salt thereof.
42. The method according to any one of embodiments 35 to 41, wherein the immune checkpoint inhibitor is an anti-PD-1/PD-L1 antibody, a PD-1/PD-L1 small molecule inhibitor, or a TIGIT inhibitor.
43. The method according to any one of embodiments 35 to 42, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
44. The use, pharmaceutical combination product or method according to any one of embodiments 35 to 42, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
45. The method according to any one of embodiments 35 to 42, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
46. The method according to any one of embodiments 35 to 38, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
47. The method according to any one of embodiments 35 to 38, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
48. The method according to any one of embodiments 35 to 38, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
49. The method according to any one of embodiments 35 to 48, wherein the FAK inhibitor, the epidermal growth factor receptor tyrosine kinase inhibitor, and the immune checkpoint inhibitor are administered to the subject simultaneously or sequentially.
50. The method according to any one of embodiments 35 to 49, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
51. The method according to any one of embodiments 35 to 50, wherein the tumor is lung cancer (including small cell lung cancer and non-small cell lung cancer) or colon cancer (including colorectal cancer), alternatively, the tumor is non-small cell lung cancer or colon cancer (including colorectal cancer).
52. An FAK inhibitor, an epidermal growth factor receptor tyrosine kinase inhibitor, and an immune checkpoint inhibitor, for use in a method of treating a tumor by increasing immunogenic cell death in a subject.
53. The FAK inhibitor, epidermal growth factor receptor tyrosine kinase inhibitor and immune checkpoint inhibitor according to embodiment 52, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 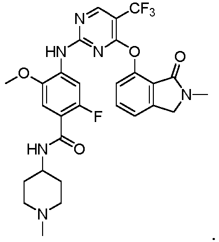
54. The FAK inhibitor, epidermal growth factor receptor tyrosine kinase inhibitor and immune checkpoint inhibitor according to embodiment 52 or 53, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
55. The FAK inhibitor, epidermal growth factor receptor tyrosine kinase inhibitor and immune checkpoint inhibitor according to any one of embodiments 52 to 54, wherein the epidermal growth factor receptor tyrosine kinase inhibitor is Osimertinib, Almonertinib, Alflutinib, or a pharmaceutically acceptable salt thereof.
56. The FAK inhibitor, epidermal growth factor receptor tyrosine kinase inhibitor and immune checkpoint inhibitor according to any one of embodiments 52 to 55, wherein the epidermal growth factor receptor tyrosine kinase inhibitor is Osimertinib or a pharmaceutically acceptable salt thereof.
57. The FAK inhibitor, epidermal growth factor receptor tyrosine kinase inhibitor and immune checkpoint inhibitor according to any one of embodiments 52 to 55, wherein the epidermal growth factor receptor tyrosine kinase inhibitor is Almonertinib or a pharmaceutically acceptable salt thereof.
58. The FAK inhibitor, epidermal growth factor receptor tyrosine kinase inhibitor and immune checkpoint inhibitor according to any one of embodiments 52 to 55, wherein the epidermal growth factor receptor tyrosine kinase inhibitor is Alflutinib or a pharmaceutically acceptable salt thereof.
59. The FAK inhibitor, epidermal growth factor receptor tyrosine kinase inhibitor and immune checkpoint inhibitor according to any one of embodiments 52 to 58, wherein the immune checkpoint inhibitor is an anti-PD-1/PD-L1 antibody, a PD-1/PD-L1 small molecule inhibitor, or a TIGIT inhibitor.
60. The FAK inhibitor, epidermal growth factor receptor tyrosine kinase inhibitor and immune checkpoint inhibitor according to any one of embodiments 52 to 59, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
61. The use, pharmaceutical combination product or method according to any one of embodiments 52 to 59, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
62. The FAK inhibitor, epidermal growth factor receptor tyrosine kinase inhibitor and immune checkpoint inhibitor according to any one of embodiments 52 to 59, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
63. The FAK inhibitor, epidermal growth factor receptor tyrosine kinase inhibitor and immune checkpoint inhibitor according to any one of embodiments 52 to 55, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
64. The FAK inhibitor, epidermal growth factor receptor tyrosine kinase inhibitor and immune checkpoint inhibitor according to any one of embodiments 52 to 55, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
65. The FAK inhibitor, epidermal growth factor receptor tyrosine kinase inhibitor and immune checkpoint inhibitor according to any one of embodiments 52 to 55, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
66. The FAK inhibitor, epidermal growth factor receptor tyrosine kinase inhibitor and immune checkpoint inhibitor according to any one of embodiments 52 to 65, wherein the FAK inhibitor, the epidermal growth factor receptor tyrosine kinase inhibitor, and the immune checkpoint inhibitor are administered to the subject simultaneously or sequentially.
67. The FAK inhibitor, epidermal growth factor receptor tyrosine kinase inhibitor and immune checkpoint inhibitor according to any one of embodiments 52 to 66, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
68. The FAK inhibitor, epidermal growth factor receptor tyrosine kinase inhibitor and immune checkpoint inhibitor according to any one of embodiments 52 to 67, wherein the tumor is lung cancer (including small cell lung cancer and non-small cell lung cancer) or colon cancer (including colorectal cancer), alternatively, the tumor is non-small cell lung cancer or colon cancer (including colorectal cancer).
69. A method of treating a tumor by increasing immunogenic cell death in a subject, wherein the method comprises administering a therapeutically effective amount of an FAK inhibitor, an epidermal growth factor receptor tyrosine kinase inhibitor, and an immune checkpoint inhibitor to the subject.
70. The method according to embodiment 69, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 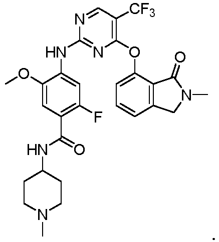
71. The method according to embodiment 69 or 70, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
72. The method according to any one of embodiments 69 to 71, wherein the epidermal growth factor receptor tyrosine kinase inhibitor is Osimertinib, Almonertinib, Alflutinib, or a pharmaceutically acceptable salt thereof.
73. The method according to any one of embodiments 69 to 72, wherein the epidermal growth factor receptor tyrosine kinase inhibitor is Osimertinib or a pharmaceutically acceptable salt thereof.
74. The method according to any one of embodiments 69 to 72, wherein the epidermal growth factor receptor tyrosine kinase inhibitor is Almonertinib or a pharmaceutically acceptable salt thereof.
75. The method according to any one of embodiments 69 to 72, wherein the epidermal growth factor receptor tyrosine kinase inhibitor is Alflutinib or a pharmaceutically acceptable salt thereof.
76. The method according to any one of embodiments 69 to 75, wherein the immune checkpoint inhibitor is an anti-PD-1/PD-L1 antibody, a PD-1/PD-L1 small molecule inhibitor, or a TIGIT inhibitor.
77. The method according to any one of embodiments 69 to 76, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
78. The use, pharmaceutical combination product or method according to any one of embodiments 69 to 76, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
79. The method according to any one of embodiments 69 to 76, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
80. The method according to any one of embodiments 69 to 72, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
81. The method according to any one of embodiments 69 to 72, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
82. The method according to any one of embodiments 69 to 72, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
83. The method according to any one of embodiments 69 to 82, wherein the FAK inhibitor, the epidermal growth factor receptor tyrosine kinase inhibitor, and the immune checkpoint inhibitor are administered to the subject simultaneously or sequentially.
84. The method according to any one of embodiments 69 to 83, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
85. The method according to any one of embodiments 69 to 84, wherein the tumor is lung cancer (including small cell lung cancer and non-small cell lung cancer) or colon cancer (including colorectal cancer), alternatively, the tumor is non-small cell lung cancer or colon cancer (including colorectal cancer).
86. Use of an FAK inhibitor in the manufacture of a medicament for treating a tumor in a subject, wherein the FAK inhibitor, an epidermal growth factor receptor tyrosine kinase inhibitor, and an immune checkpoint inhibitor are administered to the subject.
87. Use of an epidermal growth factor receptor tyrosine kinase inhibitor in the manufacture of a medicament for treating a tumor in a subject, wherein an FAK inhibitor, the epidermal growth factor receptor tyrosine kinase inhibitor and an immune checkpoint inhibitor are administered to the subject.
88. Use of an immune checkpoint inhibitor in the manufacture of a medicament for treating a tumor in a subject, wherein an FAK inhibitor, an epidermal growth factor receptor tyrosine kinase inhibitor, and the immune checkpoint inhibitor are administered to the subject.
89. Use of an FAK inhibitor, an epidermal growth factor receptor tyrosine kinase inhibitor and an immune checkpoint inhibitor in the manufacture of a combined medicament for treating a tumor.
90. Use of an FAK inhibitor in the manufacture of a combined medicament with an epidermal growth factor receptor tyrosine kinase inhibitor and an immune checkpoint inhibitor for treating a tumor.
91. Use of an epidermal growth factor receptor tyrosine kinase inhibitor in the manufacture of a combined medicament with an FAK inhibitor and an immune checkpoint inhibitor for treating a tumor.
92. Use of an immune checkpoint inhibitor in the manufacture of a combined medicament with an FAK inhibitor and an epidermal growth factor receptor tyrosine kinase inhibitor for treating a tumor.
93. Use of an FAK inhibitor, an epidermal growth factor receptor tyrosine kinase inhibitor, and an immune checkpoint inhibitor in the manufacture of a medicament for the combined treatment of a tumor.
94. Use of an FAK inhibitor in the manufacture of a medicament for the combined treatment of a tumor with an epidermal growth factor receptor tyrosine kinase inhibitor and an immune checkpoint inhibitor.
95. Use of an epidermal growth factor receptor tyrosine kinase inhibitor in the manufacture of a medicament for the combined treatment of a tumor with an FAK inhibitor and an immune checkpoint inhibitor.
96. Use of an immune checkpoint inhibitor in the manufacture of a medicament for the combined treatment of a tumor with an FAK inhibitor and an epidermal growth factor receptor tyrosine kinase inhibitor.
97. The use according to any one of embodiments 86 to 96, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows: 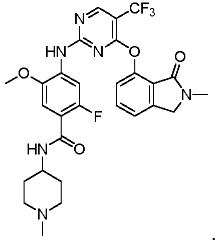
98. The use according to any one of embodiments 86 to 97, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.
99. The use according to any one of embodiments 86 to 98, wherein the epidermal growth factor receptor tyrosine kinase inhibitor is Osimertinib, Almonertinib, Alflutinib, or a pharmaceutically acceptable salt thereof.
100. The use according to any one of embodiments 86 to 99, wherein the epidermal growth factor receptor tyrosine kinase inhibitor is Osimertinib or a pharmaceutically acceptable salt thereof.
101. The use according to any one of embodiments 86 to 99, wherein the epidermal growth factor receptor tyrosine kinase inhibitor is Almonertinib or a pharmaceutically acceptable salt thereof.
102. The use according to any one of embodiments 86 to 99, wherein the epidermal growth factor receptor tyrosine kinase inhibitor is Alflutinib or a pharmaceutically acceptable salt thereof.
103. The use according to any one of embodiments 86 to 102, wherein the immune checkpoint inhibitor is an anti-PD-1/PD-L1 antibody, a PD-1/PD-L1 small molecule inhibitor, or a TIGIT inhibitor.
104. The use according to any one of embodiments 86 to 103, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.
105. The use, pharmaceutical combination product or method according to any one of embodiments 86 to 103, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.
106. The use according to any one of embodiments 86 to 103, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
107. The use according to any one of embodiments 86 to 99, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
108. The use according to any one of embodiments 86 to 99, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
109. The use according to any one of embodiments 86 to 99, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.
110. The use according to any one of embodiments 86 to 109, wherein the FAK inhibitor, the epidermal growth factor receptor tyrosine kinase inhibitor, and the immune checkpoint inhibitor are administered to the subject simultaneously or sequentially.
111. The use according to any one of embodiments 86 to 110, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
112. The use according to any one of embodiments 86 to 111, wherein the tumor is lung cancer (including small cell lung cancer and non-small cell lung cancer) or colon cancer (including colorectal cancer), alternatively, the tumor is non-small cell lung cancer or colon cancer (including colorectal cancer).

### Examples

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer. The assay materials and reagents used in the following examples are all commercially available unless otherwise specified.

The abbreviations used in the examples have the following meanings:

| Abbreviation | Meaning |
|---|---|
| siRNA | Small interfering RNA |
| DMSO | Dimethyl sulfoxide |
| COMBO | Combination |
| AAALAC | Association for Assessment and Accreditation of Laboratory Animal Care International |
| IACUC | Institutional Animal Care and Use Committee |
| SPF | Specific pathogen free |
| BIW | Twice a week |
| QD | Once a day |
| BID | Twice a day |
| BW | Body weight |
| EDTA | Ethylenediaminetetraacetic acid |
| GLP | Good Laboratory Practice for Nonclinical Laboratory Studies |
| *p*.*o*. | Oral gavage administration |
| *i.v.* | Intravenous administration |
| *i.p.* | Intraperitoneal injection administration |
| DPBS | Dulbecco's Phosphate Buffered Saline |
| DDH₂O | Double distilled water |
| RT | Room temperature |
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |
| Annexin-V-FITC | Fluorescein isothiocyanate-labeled annexin V |
| PI | Propidium iodide |

### Example 1: Study of IN10018 and AZD9291 in lung cancer KPL cells

KPL cells (Shanghai Institute of Biochemistry and Cell Biology, Chinese Academy of Sciences) were cultured with RPMI 1640 (Shanghai Basalmedia, Cat No.: L210KJ, Lot No.: F210916)+10% FBS (Gibco, Cat No.: 10099-141c, Lot No.: 2158737cp), and passaged twice. When the cells were in good condition, the culture solution was placed in a 24-well plate. After the cells were spread for 24 hours, four groups were set up, wherein the first group was a negative control group, and the culture medium was added; the second group was IN10018 with a concentration of 10 µM; the third group was AZD9291 with a concentration of 10 µM; and the fourth group was the combination group of IN10018 and AZD9291, which included IN10018 (10 µM) and AZD9291 (10 µM), respectively. The drugs were mixed, and the mixture was cultured at 37 °C in a 5% CO₂ incubator for 48 hours.

After 48 hours of drug action, the cells were observed with a microscope and photos were taken and saved. Then, the cells were collected for flow analysis, and the cells were washed twice with flow buffer (PBS+2% FBS). 0.5 µl of CoraLite^{®} 488-conjugated GRP94 polyclonal antibody (Proteintech, Cat No.: CL488-14700, Lot No.: 21006579) was added to each well and mixed. The mixture was incubated at 4 °C in the dark for 20 minutes. After 20 minutes, the cells were washed twice with flow buffer (PBS + 2% FBS). Then, the cells were analyzed on a flow cytometer.

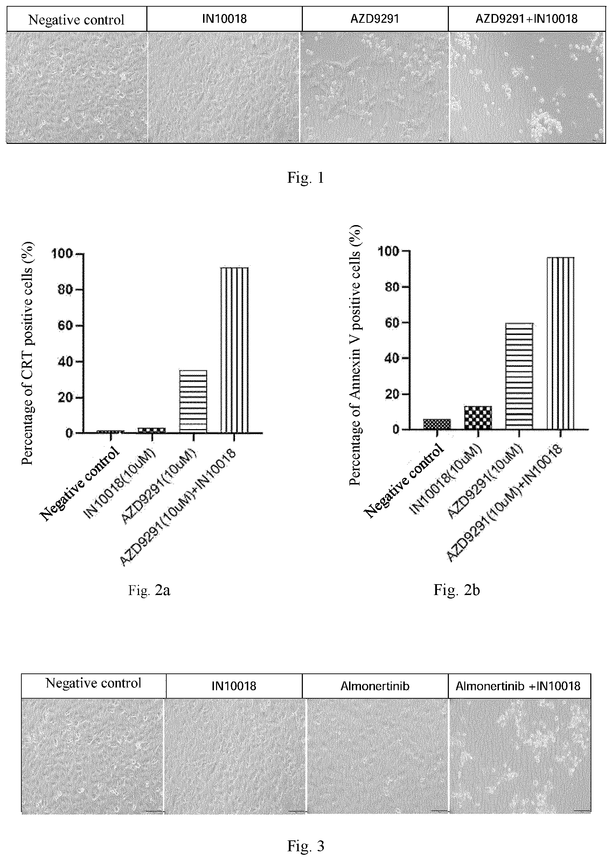

The cells were observed under a microscope. The cells of the AZD9291 single-drug group were in poor condition, and the cells of the two-drug combination group were in the worst condition, with more cell death, while the cells of the negative control group and IN10018 group were in better condition. The results of flow cytometer showed that the CRT positive rate and Annexin V positive rate of the two-drug combination group were higher than those of the single-drug group. See Fig. 1, Fig. 2a and Fig. 2b for details.

### Example 2: Study of IN10018 and Almonertinib in lung cancer KPL cells

KPL cells (Shanghai Institute of Biochemistry and Cell Biology, Chinese Academy of Sciences) were cultured with RPMI 1640 (Shanghai Basalmedia, Cat No.: L210KJ, Lot No.: F210916)+10% FBS (Gibco, Cat No.: 10099-141c, Lot No.: 2158737cp), and passaged twice. When the cells were in good condition, the culture solution was placed in a 24-well plate. After the cells were spread for 24 hours, four groups were set up, wherein the first group was a negative control group, and the culture medium was added; the second group was IN10018 with a concentration of 10 µM; the third group was Almonertinib with a concentration of 4.7 µM; and the fourth group was the combination group of IN10018 and Almonertinib, which included IN10018 (10 µM) and Almonertinib (4.7 µM), respectively. The drugs were mixed, and the mixture was cultured at 37 °C in a 5% CO₂ incubator for 48 hours.

After 48 hours of drug action, the cells were observed with a microscope and photos were taken and saved. Then, the cells were collected for flow analysis, and the cells were washed twice with flow buffer (PBS+2% FBS). 0.5 µl of CoraLite^{®} 488-conjugated GRP94 polyclonal antibody (Proteintech, Cat No.: CL488-14700, Lot No.: 21006579) was added to each well and mixed. The mixture was incubated at 4 °C in the dark for 20 minutes. After 20 minutes, the cells were washed twice with flow buffer (PBS + 2% FBS). Then, the cells were analyzed on a flow cytometer.

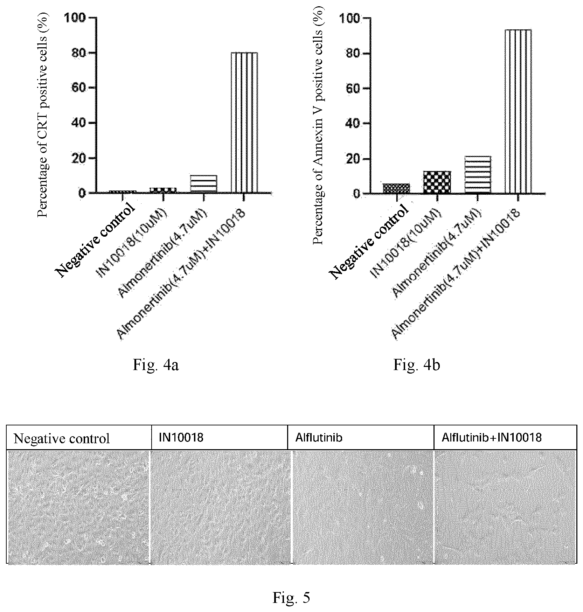

The cells were observed under a microscope. The cells of the Almonertinib single-drug group were in poor condition, and the cells of the two-drug combination group were in the worst condition, with more cell death, while the cells of the negative control group and IN10018 group were in better condition. The results of flow cytometer showed that the CRT positive rate and Annexin V positive rate of the two-drug combination group were higher than those of the single-drug group. See Fig. 3, Fig. 4a and Fig. 4b for details.

### Example 3: Study of IN10018 and Alflutinib in lung cancer KPL cells

KPL cells (Shanghai Institute of Biochemistry and Cell Biology, Chinese Academy of Sciences) were cultured with RPMI 1640 (Shanghai Basalmedia, Cat No.: L210KJ, Lot No.: F210916)+10% FBS (Gibco, Cat No.: 10099-141c, Lot No.: 2158737cp), and passaged twice. When the cells were in good condition, the culture solution was placed in a 24-well plate. After the cells were spread for 24 hours, four groups were set up, wherein the first group was a negative control group, and the culture medium was added; the second group was IN10018 with a concentration of 10 µM; the third group was Alflutinib with a concentration of 4.7 µM; and the fourth group was the combination group of IN10018 and Alflutinib, which included IN10018 (10 µM) and Alflutinib (4.7 µM), respectively. The drugs were mixed, and the mixture was cultured at 37 °C in a 5% CO₂ incubator for 48 hours.

After 48 hours of drug action, the cells were observed with a microscope and photos were taken and saved. Then, the cells were collected for flow analysis, and the cells were washed twice with flow buffer (PBS+2% FBS). 0.5 µl of CoraLite^{®} 488-conjugated GRP94 polyclonal antibody (Proteintech, Cat No.: CL488-14700, Lot No.: 21006579) was added to each well and mixed. The mixture was incubated at 4 °C in the dark for 20 minutes. After 20 minutes, the cells were washed twice with flow buffer (PBS + 2% FBS). Then, the cells were analyzed on a flow cytometer.

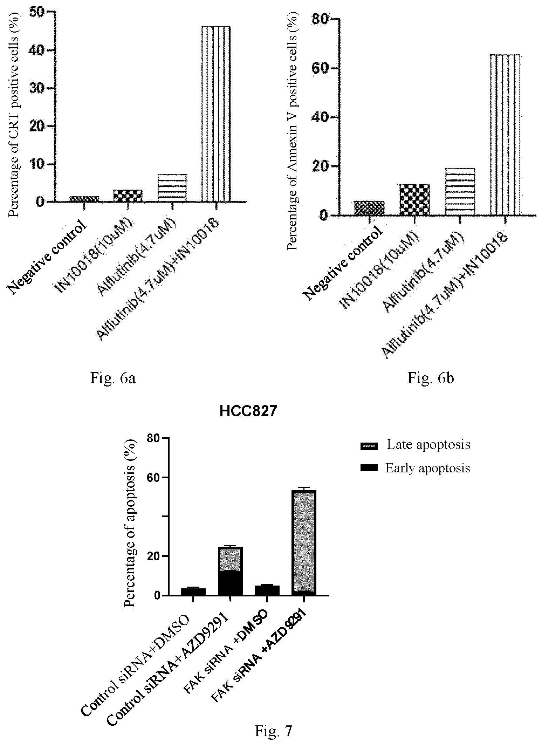

The cells were observed under a microscope. The cells of the Alflutinib single-drug group were in poor condition, and the cells of the two-drug combination group were in the worst condition, with more cell death, while the cells of the negative control group and IN10018 group were in better condition. The results of flow cytometer showed that the CRT positive rate and Annexin V positive rate of the two-drug combination group were higher than those of the single-drug group. See Fig. 5, Fig. 6a and Fig. 6b for details.

Example 4: Study on the enhancement of immunogenic cell death of non-small cell lung cancer cells in response to AZD9291 by FAK target inhibition

### Assay protocol:

1. FAK silencing combined with AZD9291 promoted apoptosis of lung cancer cells HCC827
   In an in vitro assay, siRNA technology was used to reduce the expression level of FAK, which was combined with AZD9291 for 48 hours; a flow cytometer was used to detect the apoptosis in control and FAK-silenced cells.
2. FAK silencing combined with AZD9291 can effectively promote ICD effect produced in lung cancer cells HCC827

In an in vitro assay, siRNA technology was used to reduce the expression level of FAK, which was combined with AZD9291 for 48 hours; a flow cytometer was used to detect the expression of Calreticulin, the main target of ICD.

Assay antibodies: Recombinant Alexa Fluor^{®} 647 Anti-Calreticulin antibody (Abcam, ab196159), Annexin V apoptosis detection kit (Invitrogen, A35110).

FAK siRNA was provided by GenePharma, with a sequence shown in the table below:
F: CCUGUAUGCCUAUCAGCUUTT;
R: AAGCUGAUAGGCAUACAGGTT.

### Assay instrument:

Fluorescence microscope (Olympus U-HGLGPS); Chemiluminescence imaging system (BIORAD chemidoc touch).

### Assay results:

### 1. FAK silencing combined with AZD9291 significantly increased the apoptosis of HCC827 cells

HCC827 cells were transfected with control siRNA or FAK siRNA at a final concentration of 50 nM, respectively. After 24 hours of transfection, 0.3 nM AZD9291 was added and the cells were treated for 48 hours. The cells were stained using the Annexin V kit and detected using a flow cytometer. The values of early and late apoptosis of the cells were compared with those of the DMSO control group, and Graphpad 8.0 was used for graphing.

The results showed that compared with the control group, the late apoptosis in the FAK silencing group was significantly enhanced after the treatment with AZD9291, as shown in Fig. 7.

2. FAK silencing combined with AZD9291 significantly upregulated the release and exposure of the ICD target Calreticulin.

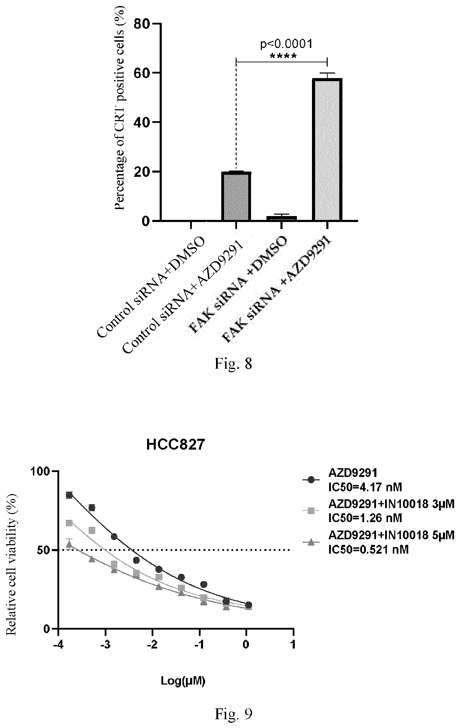

HCC827 cells were transfected with control siRNA or FAK siRNA at a final concentration of 50 nM, respectively. After 24 hours of transfection, 0.3 nM AZD9291 was added and the cells were treated for 48 hours. The Calreticulin antibody was used for fluorescent staining, and the staining results were analyzed by a flow cytometer. The statistical results of FlowJo software showed that, after FAK silencing and combined with AZD9291, the release and exposure of Calreticulin were significantly enhanced, as shown in Fig. 8.

Example 5: Study on the enhancement of immunogenic cell death of non-small cell lung cancer cells in response to AZD9291 by IN10018

### Assay protocol:

1. IN10018 combined with AZD9291 produced a synergistic tumor killing effect and induced cell apoptosis

An in vitro assay was conducted to detect the cell killing curve of AZD9291 in lung cancer cells HCC827, and the IC₅₀ value was determined. The cytotoxic effects of AZD9291 at different dosages combined with 3 µM IN10018 and 5 µM IN10018 were explored, respectively. At the same time, the Annexin V staining was used to detect the apoptotic response to the drugs used alone and in combination using a flow cytometer.

2. IN10018 combined with AZD9291 can effectively promote the ICD effect produced in lung cancer cells HCC827
In an in vitro assay, IN10018 and AZD9291 were used in combination for 6 hours, and the activation of the endoplasmic reticulum stress signaling pathway was detected; the two drugs were used in combination for 48 hours, and the expression of Calreticulin, the main target of ICD was assayed.

### Assay antibodies:

Recombinant Alexa Fluor^{®} 647 Anti-Calreticulin antibody (Abcam, ab196159), FAK antibody (CST, 3285S), Phospho-eIF2a (Ser51) (CST, 3398), eif2a (CST, 5324), DDIT3 (HUABIO, ET1703-05), HRP-conjugated alpha tubulin antibody (Proteintech, HRP-66031), Annexin V apoptosis detection kit (Invitrogen, A35110)

### Assay instrument:

Fluorescence microscope (Olympus U-HGLGPS), Chemiluminescence imaging system (BIORAD chemidoc touch).

### Assay results:

### 1. IN10018 combined with AZD9291 produced a synergistic tumor killing effect

A 96-well cell plate was plated with 5000 HCC827 cells/well. After 24 hours, IN10018 at fixed concentrations was added in combination with different concentrations of AZD9291. The concentrations of IN10018 were 3µM and 5µM; the highest concentration of AZD9291 was 1 µM and a total of 9 concentrations were set using a 3-fold concentration gradient dilution. After the drugs and cells were co-incubated for 72 hours, 10 µL of CCK8 solution was added to each well, and the plate was incubated in a 37 °C and 5% carbon dioxide incubator for 2 hours. Then, a microplate reader was used for reading with a set absorbance wavelength of 450 nm. The values read were compared with the DMSO control group, and Graphpad 8.0 was used for graphing. The results showed that AZD9291 at different dosages, combined with 3 µM IN10018 and 5 µM IN10018 for 48 hours, respectively, had a synergistic killing effect on non-small cell lung cancer cells HCC827, as shown in Fig. 9.

### 2. IN10018 combined with AZD9291 promoted cell apoptosis

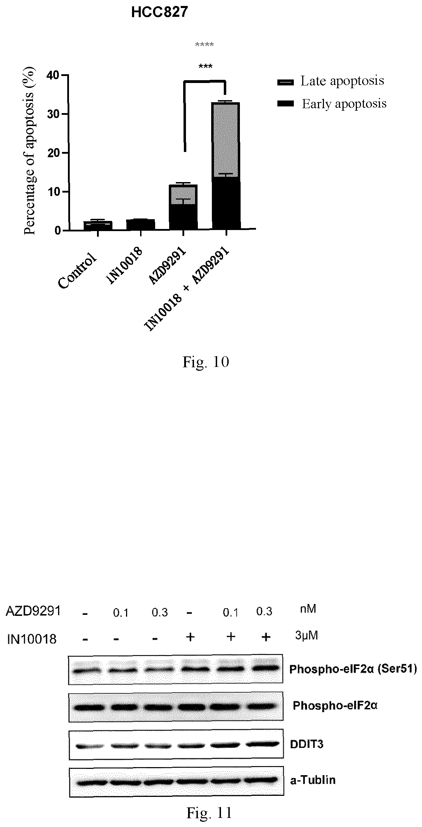

HCC827 cells were treated with 0.3 nM AZD9291 in combination with 3 µM IN10018. After 48 hours, the cells were collected, stained using Annexin V kit, and detected by a flow cytometer. The statistical values of early and late apoptosis of the cells were compared with those of the DMSO control group, and Graphpad 8.0 was used for graphing. The results showed that the early and late apoptosis in the combination group were significantly enhanced compared with the single-drug group, as shown in Fig. 10.

3. IN10018 combined with AZD9291 significantly upregulated the endoplasmic reticulum stress of HCC827
HCC827 cells were treated with 0.1 nM and 0.3 nM AZD9291 in combination with 3 µM IN10018, respectively. After 6 hours, the protein expression levels of endoplasmic reticulum stress-related proteins Phospho-eIF2a (Ser51) and DDIT3 were assayed. The results showed that, the expression levels of Phospho-eIF2a (Ser51) and DDIT3 in the combination group were significantly upregulated compared with the single-drug group, indicating that the combination of the two drugs significantly upregulated the endoplasmic reticulum stress, as shown in Fig. 11.

4. AZD9291 combined with IN10018 enhanced the release and exposure of the ICD target Calreticulin

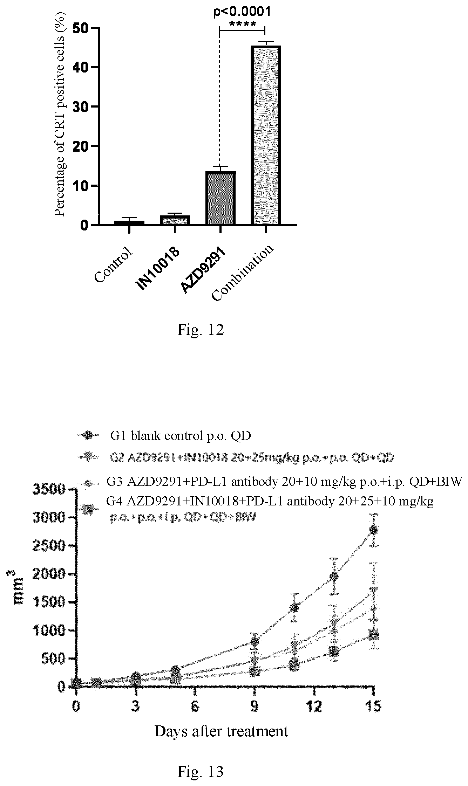

HCC827 cells were treated with 0.3 nM AZD9291 in combination with 3 µM IN10018. After 48 hours, the Calreticulin antibody was used for fluorescent staining, and the staining results were analyzed by a flow cytometer. The statistical results of FlowJo software showed that the release and exposure of Calreticulin were significantly enhanced in the combination group compared with those of the single-drug group, as shown in Fig. 12.

### Example 6: Study on the in vivo anti-tumor efficacy of AZD9291 in subcutaneous allograft tumor model of colon cancer CT26 cell in BALB/c mice

### Assay materials:

Mice: Female BALB/c mice aged 6-8 weeks were purchased from Shanghai Lingchang Biotechnology Co., Ltd. After the animals arrived, they were raised for acclimatization in the assay environment before the assay. The animals were raised in cages (5 animals per cage) with IVC (independent air supply system) in a SPF animal room. All the cages, padding and drinking water were sterilized before use. All the experimenters wore protective clothing and latex gloves when operating in the animal room. The animal information card for each cage indicated the number, sex, strain, receiving date, administration plan, assay number, group and assay start date of the animals in the cage. Cages, feed and drinking water were replaced twice a week. The feeding environment and illumination condition were as follows:
temperature: 20 to 26 °C;
humidity: 40 to 70%;
lighting period: 12 hours of light, 12 hours of dark;
cage: made of polycarbonate, with a volume of 300 mm x 180 mm x 150 mm; the padding was corncob, which was replaced twice a week;
food: assay animals can eat freely during the whole assay (radiation sterilization, dry granular food);
drinking water: assay animals can drink sterilized water freely;
animal identification: assay animals were identified by ear tags.

Information of compound is shown in Table 1

**Table 1. Information of the compound**

| **Drug name** | **Concentration (mg/mL)** | **Storage condition** | **Source** | **Solvent** | **Total amount** |
|---|---|---|---|---|---|
| AZD9291 | / | Normal temperature | Shanghai Chaolan Chemical Technology Center | 5%DMSO+40%PE G300+5%Tween-80 +50%PBS | 162 mg |
| IN10018 | / | 4 °C | It can be synthesized according to the method in patent WO2010058032 | DPBS | 135 mg |
| PDL1 antibody | 7.29 mg/mL | 4 °C | Bioxcell (BE0101) | DPBS | 15 mg |
| Polyethylene glycol 300 (PEG300) | / | Normal temperature | Sinopharm Group (30150728) | / | / |
| Dimethyl sulfoxide (DMSO) | / | Normal temperature | SIGMA (D2660) | / | / |
| Tween-80 | / | Normal temperature | Aladdin (T104866) | / | / |

Colorectal cancer cells CT26 (from Nanjing Kebai Biotechnology Co., Ltd., article number: CBP60043) were maintained and passaged by InxMed (Nanjing) Co., Ltd. The cells were cultured in monolayer in vitro, and the culture conditions were RPMI-1640 medium with 10% fetal bovine serum, 37 °C and 5% CO₂ incubator. Trypsin-EDTA was used for routine digestion and passage twice or three times a week. When the cells were in exponential growth period and the saturation reached 80% to 90%, the cells were collected, counted and inoculated.

### Cell inoculation and grouping

0.1 mL of cell suspension containing 3×10⁵ cells was subcutaneously inoculated on the right back of each mouse. When the tumor volume reached about 63 mm³ (on the 12th day after cell inoculation), mice were randomly divided into groups according to the tumor volume and administered. The grouping information is shown in Table 2.

**Table 2. Administration scheme of the assay substance in the CT26 allograft tumor model in mice**

| **Group** | **Compound for treatment** | **Number of animals¹** | **Dosage (mg/kg)** | **Dosage volume parameter (mL/kg)²** | **Route of administration** | **Frequency of administration** |
|---|---|---|---|---|---|---|
| 1 | Control group | 6 | N/A | 10 | *p.o.* | QD*15 days |
| 2 | AZD9291+IN10018 | 6 | 20+25 | 10 | *p.o.+p.o.* | QD*15 days+QD*15 days |
| 3 | AZD9291+PD-L1 antibody | 6 | 20+10 | 10 | *p.o.+i.p.* | QD*15 days + day 0, 3, 7 and 10 |
| 4 | AZD9291+IN10018 +PD-L1 antibody | 6 | 20+25+10 | 10 | *p.o.+p.o. +i.p.* | QD*15 days+QD*15 days+day 0, 3, 7 and 10 |

| | | | | | | |
|---|---|---|---|---|---|---|
| Note: 1. The number of mice in each group; | | | | | | |

2. Dosage volume: 10 mL/kg according to the body weight of mice. If the body weight loss exceeded 15%, the administration to the animal should be stopped; when the body weight returned to a loss of 10%, the administration was resumed.

### Preparation of assay substance

### See Table 3 for details

**Table 3. Preparation method of the assay substance**

| **Drug name** | **Dosage (mg/kg)** | **Concentr ation (mg/mL)** | **Preparation process** |
|---|---|---|---|
| **Control group** | / | / | 1.5 ml of DMSO, 12 ml of PEG300, 1.5 ml of Tween-80 and 15 ml of PBS were pipetted in sequence, respectively, and the mixture was vortexed to mix well. |
| (5%DMSO+40%PEG300+5% Tween-80+50%PBS) | | | |
| AZD9291 | 20 | 2 | 162 mg of AZD9291 powder was weighed, and 4.05 ml of DMSO was added. The mixture was sonicated and vortexed to mix well to give the STOCK solution. Each time being used, 1.075 ml of the STOCK solution was pipetted. 8.6 ml of PEG300, 1.075 ml of Tween-80, and 10.75 ml of PBS were added successively. The mixture was sonicated and vortexed to give a clear solution. The clear solution was prepared every 4 days. |
| PDL1 antibody | 10 | 1 | 0.48 mL of PDL1 antibody (7.29 mg/mL) was pipetted and diluted with 3.02 mL of added DPBS. The mixture was mixed well to give a clear solution. The clear solution was freshly prepared before each use. |
| IN10018 | 25 | 2.5 | 36 mg of IN10018 was weighed, and diluted with 14.4 mL of added DPBS. The mixture was mixed well to give a clear solution, which was prepared every 4 days. |

### Daily observation of the assay animals

The formulation and any modification of this assay protocol were evaluated and approved by the IACUC of ClinBridge Biotech Co. Ltd. The use and welfare of the assay animals were complied with AAALAC regulations. The health status and death of animals were monitored every day. Observing the effects of tumor growth and drug treatment on the daily behavior of animals, such as behavioral activities, food and water intake (visual inspection only), body weight change, appearance signs, or other abnormal conditions was included in the routine examination. Based on the number of animals in each group, the number of dead animals and side effects in the group were recorded.

### Termination of the assay

If the animal's health continues to deteriorate, or the tumor volume exceeds 3,000 mm³, or the animal has a serious disease or pain, the animal must be euthanized. The veterinarian should be notified and the animal should be euthanized if the following situations occur: obvious emaciation, body weight loss greater than 20%; unable to eat and drink freely; the average tumor volume in the control group reaches 2,000 mm³, and the assay will be terminated; the animal exhibits the following clinical manifestations, which continue to worsen: piloerection, hunching back, the color of ears, nose, eyes or feet turning pale, shortness of breath, convulsions, continuous diarrhea, dehydration, slow movement and vocalization.

### Tumor measurement and assay index

The diameter of a tumor was measured with a vernier caliper 3 times a week. The calculation formula of the tumor volume is: V = 0.5 × *a* × *b*², where *a* and *b* represent the long and short diameters of the tumor, respectively.

Referring to the tumor volume on the first day after grouping, the tumor growth inhibition rate TGI (%) was calculated according to the following formula.
TGI (%)=[1-(the average tumor volume of an administration group-the average tumor volume of the administration group at the beginning of treatment)/(the average tumor volume of a solvent control group-the average tumor volume of the solvent control group at the beginning of treatment)]×100%.

### Statistical analysis

Statistical analysis was performed using Prism Graphpad software based on the tumor volume and tumor weight at the end of the assay. The comparison between multiple groups was analyzed by Two-way ANOVA, and Fisher's LSD test was used for analysis. P < 0.05 was considered to have a significant difference.

### Assay results

The in vivo efficacy of the assay substance AZD9291 and/or PD-L1 antibody in combination with IN10018 in the subcutaneous allograft tumor model of mouse colorectal cancer cell CT26 in BALB/c mice was assayed. After the cell inoculation, the tumor growth was observed every day, and the animals were divided into groups according to the tumor volume on the 12th day after inoculation. The average tumor volume of the enrolled mice was about 63 mm³. Due to the tumor load, mice in the control group were euthanized on the 27th day after inoculation, i.e., on the 15th day after group administration, and the whole assay was terminated.

On the 15th day after group administration, the tumor volume of the control group was 2777.3±705.2 mm³. The tumor volumes of the AZD9291+IN10018 (20+25 mg/kg), AZD9291+PDL1 antibody (20+10 mg/kg) and AZD9291+IN10018+PDL1 antibody (20+25+10 mg/kg) treatment groups were 1693.4±1207.5 mm³, 1387.6±859.6 mm³ and 927.2±627.9 mm³, respectively. The details are shown in Table 4. Comparing the comprehensive tumor volume with the control group, the tumor growth inhibition rates (TGI) of AZD9291+IN10018 (20+25 mg/kg) group, AZD9291+PDL1 antibody (20+10 mg/kg) group and AZD9291+IN10018+PDL1 antibody (20+25+10 mg/kg) group were 40.0% (p < 0.0001), 51.2% (p < 0.0001) and 62.7% (p<0.0001), respectively. The details are shown in Table 4. Comparing the comprehensive tumor volume with the AZD9291+IN10018+PDL1 antibody (20+25+10 mg/kg) triple-drug combination group and performing the statistical analysis, the P values of the control group, AZD9291+IN10018 (20+25 mg/kg) and AZD9291+PDL1 antibody (20+10 mg/kg) treatment groups were p < 0.0001, p = 0.0032 and p = 0.0745, respectively. The tumor volumes of each dosage group at different time periods are shown in Fig. 13.

**Table 4 Evaluation of the tumor inhibition effect of the assay substance on the allograft tumor model of mouse colon cancer CT26 cell in BALB/c mice (based on the data of day 15 after group administration)**

| Group | Tumor volume on day 0 (mm³) ¹ | Tumor volume on day 15 (mm³) | TGI (%) | P value² | P value³ |
|---|---|---|---|---|---|
| Control group | 63.1±19.7 | 2777.3±705.2 | / | / | <0.0001 **** |
| AZD9291+IN10018 | 63.9±18.1 | 1693.4±1207.5 | 40.0 | <0.0001 **** | 0.0032 ** |
| AZD9291+PD-L1 antibody | 63.9±18.4 | 1387.6±859.6 | 51.2 | <0.0001 **** | 0.0745 |
| AZD9291+IN10018+ PD-L1 antibody | 63.2±16.4 | 927.2±627.9 | 62.7 | <0.0001 **** | / |

| | | | | | |
|---|---|---|---|---|---|
| Note: 1. It was calculated based on the number of days after group administration, and the data are mean±standard error of the mean. 2. ****: p<0.0001, vs. control group, Two-way ANOVA. 3. **: p<0.01, ****: p<0.0001, vs. AZD9291+IN10018+PDL1 antibody (20+25+10 mg/kg) group, Two-way ANOVA. | | | | | |

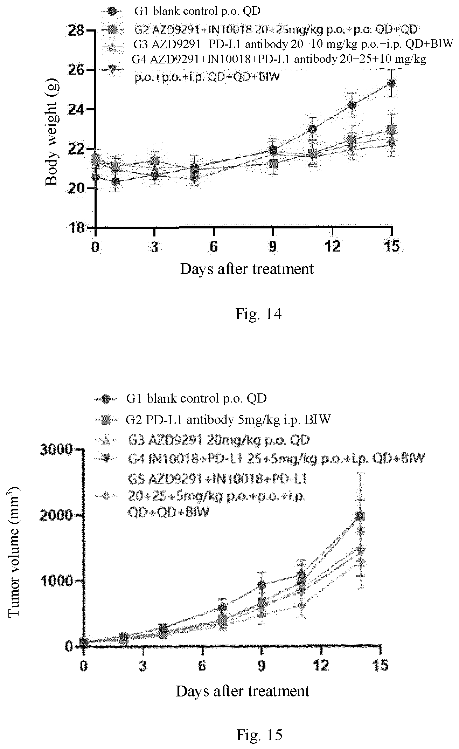

The assay was conducted according to the administration scheme. During the assay, animal activities such as eating and drinking were observed every day, and the animals' body weights were recorded 3 times a week. The body weight curves of the animals are shown in Fig. 14. During the entire administration cycle, the animals in each group showed no significant weight loss and were in good condition.

### Conclusion

Compared with the blank control group, each of the AZD9291+IN10018 (20+25 mg/kg), AZD9291+PDL1 antibody (20+10 mg/kg) and AZD9291+IN10018+PDL1 antibody (20+25+10 mg/kg) treatment groups had a significant tumor growth inhibition effect, which was statistically different from the control group. Comprehensively considering the entire administration cycle, the tumor volume of the AZD9291+IN10018+PDL1 antibody (20+25+10 mg/kg) triple-drug combination group was always smaller than that of the AZD9291+IN10018 (20+25 mg/kg) and AZD9291+PDL1 antibody (20+10 mg/kg) two-drug combination groups, and there was a statistical difference compared with the AZD9291+IN10018 (20+25 mg/kg) group. Compared with the AZD9291+IN10018 (20+25 mg/kg) and AZD9291+PDL1 antibody (20+10 mg/kg) two-drug combination groups, the triple-drug combination group had a better effect in inhibiting tumor growth. At the same time, the animals' body weights changed well, and no abnormalities were found in their activities, drinking water, eating, and mental state during the entire administration cycle, indicating that the animals were tolerant to the triple-drug combination of AZD9291+IN10018+PDL1 antibody (20+25+10 mg/kg).

### Example 7: Study on the in vivo anti-tumor efficacy of AZD9291 in subcutaneous allograft tumor model of colon cancer MC38 cell in C57BL/6 mice

### Assay materials:

Mice: Female C57BL/6 mice aged 6-8 weeks were purchased from Shanghai Slack Laboratory Animal Co., Ltd. After the animals arrived, they were raised for acclimatization in the assay environment before the assay. The animals were raised in cages (5 animals per cage) with IVC (independent air supply system) in a SPF animal room. All the cages, padding and drinking water were sterilized before use. All the experimenters wore protective clothing and latex gloves when operating in the animal room. The animal information card for each cage indicated the number, sex, strain, receiving date, administration plan, assay number, group and assay start date of the animals in the cage. Cages, feed and drinking water were replaced twice a week. The feeding environment and illumination condition were as follows:
temperature: 20 to 26 °C;
humidity: 40 to 70%;
lighting period: 12 hours of light, 12 hours of dark;
cage: made of polycarbonate, with a volume of 300 mm x 180 mm x 150 mm; the padding was corncob, which was replaced twice a week;
food: assay animals can eat freely during the whole assay (radiation sterilization, dry granular food);
drinking water: assay animals can drink sterilized water freely;
animal identification: assay animals were identified by ear tags.

Information of compound is shown in Table 5.

**Table 5. Information of the compound**

| **Drug name** | **Concentration (mg/mL)** | **Storage condition** | **Source** | **Solvent** | **Total amount** |
|---|---|---|---|---|---|
| AZD9291 | / | Room temperature | Shanghai Chaolan Chemical Technology Center | 5%DMSO+40%PEG 300+5%Tween-80+5 0%PBS | 100 mg |
| IN10018 | / | 4 °C | It can be synthesized according to the method in patent WO2010058032 | DPBS | 130 mg |
| PDL1 antibody | 7.29 mg/mL | 4 °C | Bioxcell (BE0101) | DPBS | 13.5mg |
| Polyethylene glycol 300 (PEG300) | / | Normal temperature | Sinopharm Group (30150728) | / | / |
| Dimethyl sulfoxide (DMSO) | / | Normal temperature | SIGMA (D2660) | / | / |
| Tween-80 | / | Normal temperature | Aladdin (T104866) | / | / |

Colorectal cancer cells MC38 (from Nanjing Kebai Biotechnology Co., Ltd., article number: CBP60825) were maintained and passaged by InxMed (Nanjing) Co., Ltd. The cells were cultured in monolayer in vitro, and the culture conditions were DMEM medium with 10% fetal bovine serum, 37 °C and 5% CO₂ incubator. Trypsin-EDTA was used for routine digestion and passage twice or three times a week. When the cells were in exponential growth period and the saturation reached 80% to 90%, the cells were collected, counted and inoculated.

### Cell inoculation and grouping

0.1 mL of cell suspension containing 2×10⁵ cells was subcutaneously inoculated on the right back of each mouse. When the tumor volume reached about 70 mm³ (on the 15th day after cell inoculation), mice were randomly divided into groups according to the tumor volume and administered. The grouping information is shown in Table 6.

**Table 6. Administration scheme of the assay substance in the MC38 allograft tumor model in mice**

| **Group** | **Compound for treatment** | **Number of animals¹** | **Dosage (mg/kg)** | **Dosage volume parameter (mL/kg)²** | **Route of administra tion** | **Frequency of administration** |
|---|---|---|---|---|---|---|
| 1 | Control group | 6 | N/A | 10 | *p.o.* | QD*2 weeks |
| 2 | PD-L1 antibody | 6 | 5 | 10 | *i.p.* | BIW*2 weeks |
| 3 | AZD9291 | 6 | 20 | 10 | *p.o.* | QD*2 weeks |
| 4 | IN10018+PD-L1 antibody | 6 | 25+5 | 10 | *p.o.+i.p.* | QD*2 weeks+BIW*2 weeks |
| 5 | AZD9291+IN1 0018+PD-L1 antibody | 6 | 20+25+5 | 10 | *p.o.+p.o.+i. p.* | QD*16 days+QD*16 days+BIW*2 weeks |

| | | | | | | |
|---|---|---|---|---|---|---|
| Note: 1. The number of mice in each group; | | | | | | |

2. Dosage volume: 10 mL/kg according to the body weight of mice. If the body weight loss exceeded 15%, the administration to the animal should be stopped; when the body weight returned to a loss of 10%, the administration was resumed.

### Preparation of assay substance

### See Table 7 for details

**Table 7. Preparation method of the assay substance**

| **Drug name** | **Dosage (mg/kg)** | **Concentr ation (mg/mL)** | **Preparation process** |
|---|---|---|---|
| **Control group** | / | / | 1.5 ml of DMSO, 12 ml of PEG300, 1.5 ml of Tween-80 and 15 ml of PBS were pipetted in sequence, respectively, and the mixture was vortexed to mix well. |
| (5%DMSO+40%PEG300+5%T ween-80+50%PBS) | | | |
| **AZD9291** | 20 | 2 | 100 mg of AZD9291 powder was weighed, and 2.5 ml of DMSO was added. The mixture was sonicated and vortexed to mix well to give the STOCK solution. Each time being used, 0.6 ml of the STOCK solution was pipetted. 4.8 ml of PEG300, 0.6 ml of Tween-80, and 6.0 ml of PBS were added successively. The mixture was sonicated and vortexed to give a clear solution. The clear solution was prepared every 4 days. |
| **IN10018** | 25 | 2.5 | 32.5 mg of IN10018 was weighed, and diluted with 13.0 mL of added DPBS. The mixture was mixed well to give a clear solution, which was prepared every 4 days. |
| **PD-L1 antibody** | 5 | 0.5 | 0.463 mL of PDL1 antibody (7.29 mg/mL) was pipetted and diluted with 6.287 mL of added DPBS. The mixture was mixed well to give a clear solution. The clear solution was freshly prepared before each use. |

### Daily observation of the assay animals

The formulation and any modification of this assay protocol were evaluated and approved by the IACUC of ClinBridge Biotech Co. Ltd. The use and welfare of the assay animals were complied with AAALAC regulations. The health status and death of animals were monitored every day. Observing the effects of tumor growth and drug treatment on the daily behavior of animals, such as behavioral activities, food and water intake (visual inspection only), body weight change, appearance signs, or other abnormal conditions was included in the routine examination. Based on the number of animals in each group, the number of dead animals and side effects in the group were recorded.

### Termination of the assay

If the animal's health continues to deteriorate, or the tumor volume exceeds 3,000 mm³, or the animal has a serious disease or pain, the animal must be euthanized. The veterinarian should be notified and the animal should be euthanized if the following situations occur: obvious emaciation, body weight loss greater than 20%; unable to eat and drink freely; the average tumor volume in the control group reaches 2,000 mm³, and the assay will be terminated; the animal exhibits the following clinical manifestations and continues to worsen: piloerection, hunching back, the color of ears, nose, eyes or feet turning pale, shortness of breath, convulsions, continuous diarrhea, dehydration, slow movement and vocalization.

### Tumor measurement and assay index

The diameter of a tumor was measured with a vernier caliper 3 times a week. The calculation formula of the tumor volume is: V = 0.5 × *a* × *b*², where *a* and *b* represent the long and short diameters of the tumor, respectively.

Referring to the tumor volume on the first day after grouping, the tumor growth inhibition rate TGI (%) was calculated according to the following formula. TGI (%)=[1-(the average tumor volume of an administration group-the average tumor volume of the administration group at the beginning of treatment)/(the average tumor volume of a solvent control group-the average tumor volume of the solvent control group at the beginning of treatment)]×100%.

### Statistical analysis

Statistical analysis was performed using Prism Graphpad software based on the tumor volume and tumor weight at the end of the assay. The comparison between multiple groups was analyzed by Two-way ANOVA, and Fisher's LSD test was used for analysis. P < 0.05 was considered to have a significant difference.

### Assay results

The in vivo efficacy of the assay substance AZD9291 and IN10018 in combination with PD-L1 antibody in the subcutaneous allograft tumor model of mouse colorectal cancer cell MC38 in C57BL/6 mice was assayed. After the cell inoculation, the tumor growth was observed every day, and the animals were divided into groups according to the tumor volume on the 15th day after inoculation. The average tumor volume of the enrolled mice was about 70 mm³. Due to the tumor load, mice in the control group were euthanized on the 29th day after inoculation, i.e., on the 14th day after group administration, and the whole assay was terminated.

On the 14th day after group administration, the tumor volume of the control group was 1984.4±537.8 mm³. The tumor volumes of the PD-L1 antibody (5 mg/kg), AZD9291 (20 mg/kg), IN10018+PD-L1 antibody (25+5 mg/kg), and AZD9291+IN10018+PD-L1 antibody (20+25+5 mg/kg) treatment groups were 1974.8±1629.9 mm³, 1518.1±728.8 mm³, 1425.2±889.6 mm³ and 1292.9±934.6 mm³, respectively. The details are shown in Table 8. Comparing the comprehensive tumor volume with the control group, the tumor growth inhibition rates (TGI) of PD-L1 antibody (5 mg/kg) group, AZD9291 (20 mg/kg) group, IN10018+PD-L1 antibody (25+5 mg/kg) group, and AZD9291+IN10018+PD-L1 antibody (20+25+5 mg/kg) group were 0.5 % (p = 0.9728), 24.4 % (p = 0.1012), 29.2 % (p = 0.0498) and 36.1 % (p = 0.0205), respectively. The details are shown in Table 8. Comparing the comprehensive tumor volume with the AZD9291+IN10018+PD-L1 antibody (20+25+5 mg/kg) combination group and performing the statistical analysis, the P values of the control group, PD-L1 antibody (5 mg/kg), AZD9291 (20 mg/kg), and IN10018+PD-L1 antibody (25+5 mg/kg) groups were p = 0.0205, p = 0.0170, p = 0.4274 and p = 0.6408, respectively. The tumor volumes of each dosage group at different time periods are shown in Fig. 15.

**Table 8 Evaluation of the tumor inhibition effect of the assay substance on the allograft tumor model of mouse colon cancer MC38 cell in C57BL/6 mice (based on the data of day 14 after group administration)**

| **Group** | **Tumor volume on day 0 (mm³) ¹** | **Tumor volume on day 14 (mm³)** | **TGI (%)** | **P value²** | **P value³** |
|---|---|---|---|---|---|
| Control group | 70.3±34.8 | 1984.4±537.8 | / | / | 0.0205 * |
| PD-L1 antibody | 70.6±32.3 | 1974.8±1629.9 | 0.5 | 0.9728 | 0.0170 * |
| AZD9291 | 71.0±24.8 | 1518.1±728.8 | 24.4 | 0.1012 | 0.4274 |
| IN10018+PD-L1 antibody | 70.7±25.9 | 1425.2±889.6 | 29.2 | 0.0498 * | 0.6408 |
| AZD9291+IN10018 +PD-L1 antibody | 70.6±26.1 | 1292.9±934.6 | 36.1 | 0.0205 * | / |

| | | | | | |
|---|---|---|---|---|---|
| Note: 1. It was calculated based on the number of days after group administration, and the data are mean±standard error of the mean. 2. *: p<0.05, vs. control group, Two-way ANOVA. 3. *: p<0.05, vs. AZD9291+IN10018+PD-L1 antibody (20+25+5 mg/kg) group, Two-way ANOVA. | | | | | |

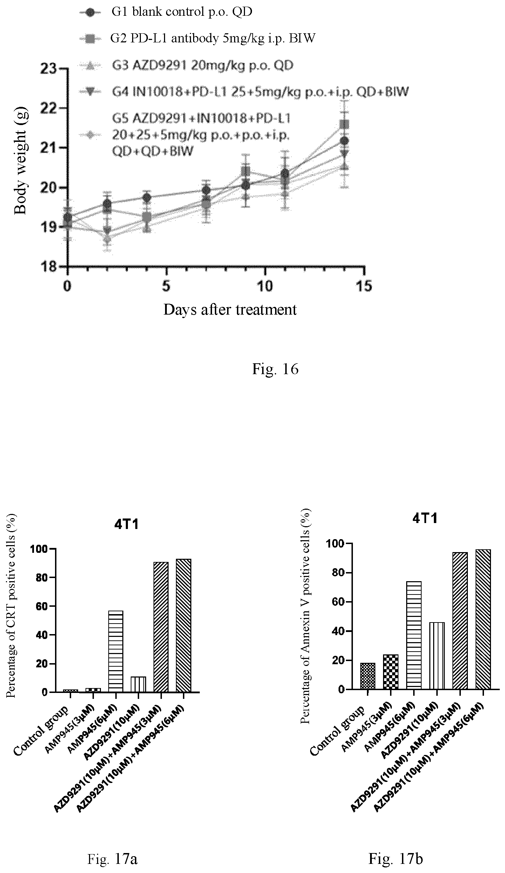

The assay was conducted according to the administration scheme. During the assay, animal activities such as eating and drinking were observed every day, and the animals' body weights were recorded 3 times a week. The body weight curves of the animals are shown in Fig. 16. There was one mouse in the control group, which was found dead on the 11th day after group administration due to tumor rupture; there was one mouse in the AZD9291+IN10018+PD-L1 antibody (20+25+5 mg/kg) group, which died on the 8th day after group administration due to an error in oral gavage administration. The animals in the other groups showed no significant weight loss and were in good condition throughout the entire administration cycle, showing tolerance to different administration methods.

### Conclusion

Compared with the blank control group, each of the IN10018+PD-L1 antibody (25+5 mg/kg) and AZD9291+IN10018+PD-L1 antibody (20+25+5 mg/kg) groups had a significant tumor growth inhibition effect, which was statistically different from the control group. Comprehensively considering the entire administration cycle, the tumor volume of the AZD9291+IN10018+PD-L1 antibody (20+25+5 mg/kg) group was always smaller than that of each of the other treatment groups, and there was a statistical difference compared with the PD-L1 antibody (5 mg/kg) single-drug group. Although there was no statistical difference between the triple-drug combination group and the AZD9291 (20 mg/kg) group and IN10018+PD-L1 antibody (25+5 mg/kg) group, the average tumor volume of the triple-drug combination group was always smaller than that of each of the other treatment groups, which also showed that the AZD9291+IN10018+PD-L1 antibody (20+25+5 mg/kg) triple-drug combination group had a better effect in inhibiting tumor growth. At the same time, except for the animals that died naturally due to tumor rupture and the operation error by humans, the weights of the other animals in each group changed well, and no abnormalities were found in the activities, drinking water, eating, and mental state during the entire administration cycle, indicating that the animals were tolerant to the triple-drug combination of AZD9291+IN10018+PD-L1 antibody (20+25+5 mg/kg).

### Example 8: Study on the induction of immunogenic cell death targets of mouse breast cancer 4T1 cells in vitro by AZD9291 and AMP945

### Assay materials:

### 1) Drugs used in this assay

AZD9291 was provided by Shanghai Chaolan Chemical Technology Center, Lot No.: 202012-02
AMP945 was provided by MCE, Lot No.: 143253

### 2) Antibodies used in this assay

Recombinant Alexa Fluor^{®} 647 Anti-Calreticulin antibody (Abcam, Cat No.: ab196159, Lot No.: CR33676773); Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp). The culture conditions were 37 °C and 5% CO₂. Trypsin was used for routine digestion and passage treatment twice or three times a week. When the cells were in exponential growth period and the confluency of the adherent cells reached 80% to 90%, the cells were collected and plated. The 4T1 cells were digested with trypsin, and then collected and counted. According to the counting results, the cells were diluted with RPM11640+10%FBS, and the dilution concentration was 50,000 cells/ml. Then, the cells were plated on a 12-well cell culture plate, and 2 ml of cell suspension (100,000 cells) was plated in each well. After the plating, cells were cultured in a 37 °C and 5% CO₂ incubator. 24 hours after the cells were spread, six groups were set up, wherein the first group was a control group, and the culture medium was added; the second group was AMP945 with a concentration of 3µM; the third group was AMP945 with a concentration of 6 µM; the fourth group was AZD9291 with a concentration of 10 µM; the fifth group was AMP945 (3 µM) combined with AZD9291 (10 µM); and the sixth group was AMP945(6 µM) combined with AZD9291 (10 µM). The drugs were mixed, and the mixture was cultured at 37 °C in a 5%CO₂ incubator for 48 hours.

### Assay results

After 48 hours of drug action, the cells were collected for flow analysis. The cells were washed twice with flow buffer (PBS+2%FBS) and 0.5 µl of AF647. An anti-Calreticulin antibody (abcam) was added to each well and mixed. The mixture was incubated at 4 °C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination. The results of flow cytometry analysis showed that the CRT positive rate and Annexin-V positive rate of the two-drug combination groups were significantly better than those of the single-drug groups and the control group, which are shown in Fig. 17a and Fig. 17b.

### Example 9: Study on the induction of immunogenic cell death targets of mouse breast cancer 4T1 cells in vitro by Alflutinib and AMP945

### Assay materials:

### 1) Drugs used in this assay

Alflutinib was provided by Shanghai MCE, Lot No.: 33805
AMP945 was provided by MCE, Lot No.: 143253

### 2) Antibodies used in this assay

Recombinant Alexa Fluor^{®} 647 Anti-Calreticulin antibody (Abcam, Cat No.: ab196159, Lot No.: CR33676773); Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp). The culture conditions were 37 °C and 5% CO₂. Trypsin was used for routine digestion and passage treatment twice or three times a week. When the cells were in exponential growth period and the confluency of the adherent cells reached 80% to 90%, the cells were collected and plated. The 4T1 cells were digested with trypsin, and then collected and counted. According to the counting results, the cells were diluted with RPM11640+10%FBS, and the dilution concentration was 50,000 cells/ml. Then, the cells were plated on a 12-well cell culture plate, and 2 ml of cell suspension (100,000 cells) was plated in each well. After the plating, cells were cultured in a 37 °C and 5% CO₂ incubator. 24 hours after the cells were spread, six groups were set up, wherein the first group was a control group, and the culture medium was added; the second group was AMP945 with a concentration of 3µM; the third group was AMP945 with a concentration of 6 µM; the fourth group was Alflutinib with a concentration of 10 µM; the fifth group was AMP945 (3 µM) combined with Alflutinib (10 µM); and the sixth group was AMP945(6 µM) combined with Alflutinib (10 µM). The drugs were mixed, and the mixture was cultured at 37 °C in a 5%CO₂ incubator for 48 hours.

### Assay results

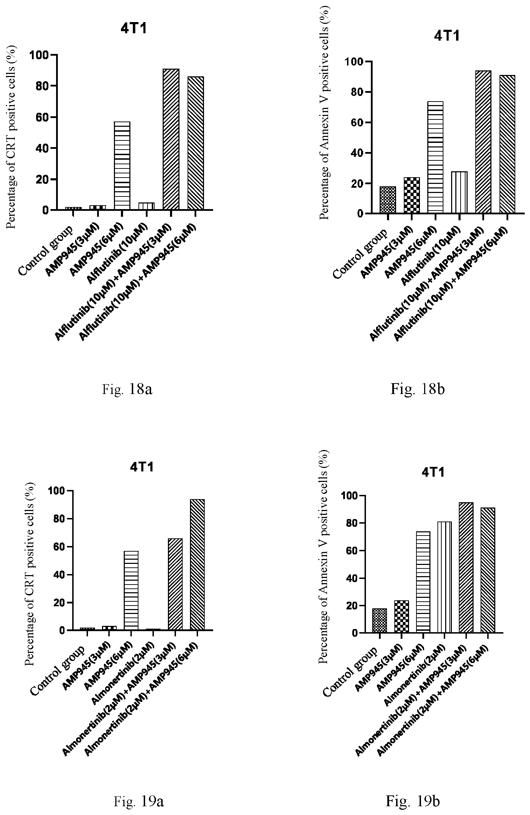

After 48 hours of drug action, the cells were collected for flow analysis. The cells were washed twice with flow buffer (PBS+2%FBS) and 0.5 µl of AF647. An anti-Calreticulin antibody (abcam) was added to each well and mixed. The mixture was incubated at 4 °C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination. The results of flow cytometry analysis showed that the CRT positive rate and Annexin-V positive rate of the two-drug combination groups were significantly better than those of the single-drug groups and the control group, which are shown in Fig. 18a and Fig. 18b.

### Example 10: Study on the induction of immunogenic cell death targets of mouse breast cancer 4T1 cells in vitro by Almonertinib and AMP945

### Assay materials:

### 1) Drugs used in this assay

Almonertinib was provided by Shanghai MCE, Lot No.: 65966
AMP945 was provided by MCE, Lot No.: 143253

### 2) Antibodies used in this assay

Recombinant Alexa Fluor^{®} 647 Anti-Calreticulin antibody (Abcam, Cat No.: ab196159, Lot No.: CR33676773); Annexin V-apoptosis detection kit (Beyotime, Cat No.: C1062L, Lot No.: 122221220706).

### Assay method:

4T1 cells (from Nanjing Kebai Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp). The culture conditions were 37 °C and 5% CO₂. Trypsin was used for routine digestion and passage treatment twice or three times a week. When the cells were in exponential growth period and the confluency of the adherent cells reached 80% to 90%, the cells were collected and plated. The 4T1 cells were digested with trypsin, and then collected and counted. According to the counting results, the cells were diluted with RPM11640+10%FBS, and the dilution concentration was 50,000 cells/ml. Then, the cells were plated on a 12-well cell culture plate, and 2 ml of cell suspension (100,000 cells) was plated in each well. After the plating, cells were cultured in a 37 °C and 5% CO₂ incubator. 24 hours after the cells were spread, six groups were set up, wherein the first group was a control group, and the culture medium was added; the second group was AMP945 with a concentration of 3µM; the third group was AMP945 with a concentration of 6 µM; the fourth group was Almonertinib with a concentration of 2 µM; the fifth group was AMP945 (3 µM) combined with Almonertinib (2 µM); and the sixth group was AMP945(6 µM) combined with Almonertinib (2 µM). The drugs were mixed, and the mixture was cultured at 37 °C in a 5% CO₂ incubator for 48 hours.

### Assay results

After 48 hours of drug action, the cells were collected for flow analysis. The cells were washed twice with flow buffer (PBS+2%FBS) and 0.5 µl of AF647. An anti-Calreticulin antibody (abcam) was added to each well and mixed. The mixture was incubated at 4 °C in the dark. After 20 minutes of incubation, the flow buffer was added. An Annexin staining kit (Beyotime) was used, and 195 µl of Annexin-V-FITC conjugate was added and mixed with the cells by pipetting. Then, 5 µl of Annexin-V-FITC antibody was added and gently mixed. Finally, 10 µl of PI dye was added and mixed. The mixture was incubated at room temperature in the dark for 15 min. A sample was sent to a flow cytometer for signal determination. The results of flow cytometry analysis showed that the CRT positive rate and Annexin-V positive rate of the two-drug combination groups were significantly better than those of the single-drug groups and the control group, which are shown in Fig. 19a and Fig. 19b.

All references mentioned in the present disclosure are incorporated by reference in their entirety as if each document was listed separately. It should be understood that after reading the present disclosure, those skilled in the art can make various changes or modifications to the present disclosure, and these equivalents also fall within the scope defined by the claims of this application.

## Claims

1. Use of an FAK inhibitor, an epidermal growth factor receptor tyrosine kinase inhibitor and an immune checkpoint inhibitor in the manufacture of a medicament for treating a tumor in a subject.

2. A pharmaceutical combination product of an FAK inhibitor, an epidermal growth factor receptor tyrosine kinase inhibitor and an immune checkpoint inhibitor, for use in treating a tumor in a subject.

3. A method of treating a tumor, comprising administering a therapeutically effective amount of an FAK inhibitor, an epidermal growth factor receptor tyrosine kinase inhibitor and an immune checkpoint inhibitor to a subject.

4. The use, the pharmaceutical combination product, or the method according to any one of claims 1 to 3, wherein the FAK inhibitor and the epidermal growth factor receptor tyrosine kinase inhibitor induce immunogenic cell death (ICD).

5. The use, the pharmaceutical combination product, or the method according to any one of claims 1 to 4, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows:

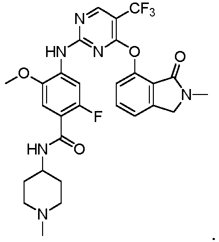

6. The use, the pharmaceutical combination product, or the method according to any one of claims 1 to 5, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.

7. The use, the pharmaceutical combination product, or the method according to any one of claims 1 to 6, wherein the epidermal growth factor receptor tyrosine kinase inhibitor is Osimertinib, Almonertinib, Alflutinib, or a pharmaceutically acceptable salt thereof.

8. The use, the pharmaceutical combination product, or the method according to any one of claims 1 to 7, wherein the epidermal growth factor receptor tyrosine kinase inhibitor is Osimertinib or a pharmaceutically acceptable salt thereof.

9. The use, the pharmaceutical combination product, or the method according to any one of claims 1 to 8, wherein the epidermal growth factor receptor tyrosine kinase inhibitor is Almonertinib or a pharmaceutically acceptable salt thereof.

10. The use, the pharmaceutical combination product, or the method according to any one of claims 1 to 8, wherein the epidermal growth factor receptor tyrosine kinase inhibitor is Alflutinib or a pharmaceutically acceptable salt thereof.

11. The use, the pharmaceutical combination product, or the method according to any one of claims 1 to 10, wherein the immune checkpoint inhibitor is an anti-PD-1/PD-L1 antibody, a PD-1/PD-L1 small molecule inhibitor, or a TIGIT inhibitor.

12. The use, the pharmaceutical combination product, or the method according to any one of claims 1 to 11, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.

13. The use, the pharmaceutical combination product, or the method according to any one of claims 1 to 11, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.

14. The use, the pharmaceutical combination product, or the method according to any one of claims 1 to 11, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

15. The use, the pharmaceutical combination product, or the method according to any one of claims 1 to 4, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

16. The use, the pharmaceutical combination product, or the method according to any one of claims 1 to 4, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

17. The use, the pharmaceutical combination product, or the method according to any one of claims 1 to 4, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

18. The use, the pharmaceutical combination product, or the method according to any one of claims 1 to 17, wherein the FAK inhibitor, the epidermal growth factor receptor tyrosine kinase inhibitor and the immune checkpoint inhibitor are administered to the subject simultaneously or sequentially.

19. The use, the pharmaceutical combination product, or the method according to any one of claims 1 to 18, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.

20. The use, the pharmaceutical combination product, or the method according to any one of claims 1 to 19, wherein the tumor is lung cancer (including small cell lung cancer and non-small cell lung cancer) or colon cancer (including colorectal cancer), alternatively, the tumor is non-small cell lung cancer or colon cancer (including colorectal cancer).

21. A kit or a pharmaceutically acceptable composition, comprising:
(a) an FAK inhibitor;
(b) an epidermal growth factor receptor tyrosine kinase inhibitor; and
(c) an immune checkpoint inhibitor.

22. The kit or composition according to claim 21, wherein the FAK inhibitor and the epidermal growth factor receptor tyrosine kinase inhibitor induce immunogenic cell death (ICD).

23. The kit or composition according to claim 21 or 22, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows:

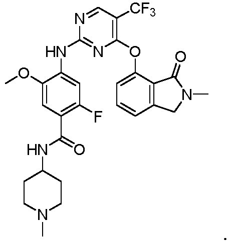

24. The kit or composition according to any one of claims 21 to 23, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof.

25. The kit or composition according to any one of claims 21 to 24, wherein the epidermal growth factor receptor tyrosine kinase inhibitor is Osimertinib, Almonertinib, Alflutinib, or a pharmaceutically acceptable salt thereof.

26. The kit or composition according to any one of claims 21 to 25, wherein the epidermal growth factor receptor tyrosine kinase inhibitor is Osimertinib or a pharmaceutically acceptable salt thereof.

27. The kit or composition according to any one of claims 21 to 25, wherein the epidermal growth factor receptor tyrosine kinase inhibitor is Almonertinib or a pharmaceutically acceptable salt thereof.

28. The kit or composition according to any one of claims 21 to 25, wherein the epidermal growth factor receptor tyrosine kinase inhibitor is Alflutinib or a pharmaceutically acceptable salt thereof.

29. The kit or composition according to any one of claims 21 to 28, wherein the immune checkpoint inhibitor is an anti-PD-1/PD-L1 antibody, a PD-1/PD-L1 small molecule inhibitor, or a TIGIT inhibitor.

30. The kit or composition according to any one of claims 21 to 29, wherein the immune checkpoint inhibitor is an anti-PD-1/PD-L1 antibody, and further, the anti-PD-1/PD-L1 antibody is Pembrolizumab, Tislelizumab, Nivolumab, Toripalimab, Atezolizumab, Durvalumab, Avelumab, Camrelizumab, Sintilimab, Cemiplimab, Envafolimab, BMS-936559, JS003, SHR-1316, GS-4224, AN-4005, or MX-10181.

31. The kit or composition according to any one of claims 21 to 29, wherein the immune checkpoint inhibitor is a PD-1/PD-L1 small molecule inhibitor, and further, the PD-1/PD-L1 small molecule inhibitor is INCB-086550, Lazertinib, IMMH-010, CA-170, ABSK043, or RRx-001.

32. The kit or composition according to any one of claims 21 to 29, wherein the immune checkpoint inhibitor is a TIGIT inhibitor, and further, the TIGIT inhibitor is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

33. The kit or composition according to claim 21 or 22, wherein the FAK inhibitor is IN10018 or a pharmaceutically acceptable salt thereof, the epidermal growth factor receptor tyrosine kinase inhibitor is Osimertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

34. The kit or composition according to claim 21 or 22, wherein the FAK inhibitor is IN10018 or a pharmaceutically acceptable salt thereof, the epidermal growth factor receptor tyrosine kinase inhibitor is Almonertinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

35. The kit or composition according to claim 21 or 22, wherein the FAK inhibitor is IN10018 or a pharmaceutically acceptable salt thereof, the epidermal growth factor receptor tyrosine kinase inhibitor is Alflutinib or a pharmaceutically acceptable salt thereof; and the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

36. The kit or composition according to any one of claims 21 to 35, for use as a medicament.

37. The kit or composition according to any one of claims 21 to 36, wherein the medicament is used in treating a tumor, and the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.

38. The kit or composition according to claim 37, wherein the tumor is lung cancer (including small cell lung cancer and non-small cell lung cancer) or colon cancer (including colorectal cancer), alternatively, the tumor is non-small cell lung cancer or colon cancer (including colorectal cancer).

39. An FAK inhibitor, for use in enhancing immunogenic cell death induced by an epidermal growth factor receptor tyrosine kinase inhibitor in the treatment of a tumor.

40. The FAK inhibitor according to claim 39, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, AMP945, Defactinib, or a pharmaceutically acceptable salt thereof, yet alternatively, the FAK inhibitor is IN10018 or a pharmaceutically acceptable salt thereof, alternatively, the FAK inhibitor is a tartrate of IN10018, and structure of the IN10018 is as follows:

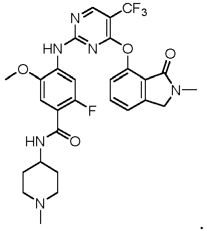

41. The FAK inhibitor according to claim 39 or 40, wherein the epidermal growth factor receptor tyrosine kinase inhibitor is Gefitinib, Erlotinib, Icotinib, Afatinib, Dacomitinib, Crizotinib, Osimertinib (AZD9291), Almonertinib, Alflutinib (also known as Furmonertinib), EAI045, JBJ-04-125-02, BLU-945, BLU-701, TQB3804, BBT-176, ES-072, BPI-361175, CH7233163, or a pharmaceutically acceptable salt thereof; alternatively, the epidermal growth factor receptor tyrosine kinase inhibitor is Osimertinib, Almonertinib, Alflutinib, or a pharmaceutically acceptable salt thereof.

42. The FAK inhibitor according to any one of claims 39 to 41, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine cancer, ovarian cancer, salivary gland cancer, metastases from spindle cell carcinoma, anaplastic large cell lymphoma, anaplastic thyroid cancer, non-Hodgkin lymphoma, Hodgkin lymphoma, glioma or hematological malignancies such as acute myeloid leukemia (AML), acute lymphoblastic leukemia (ALL), diffuse large B-cell lymphoma (DLBCL), follicular lymphoma (FL), chronic lymphocytic leukemia (CLL), and chronic myeloid leukemia (CML); alternatively, the tumor is breast cancer, ovarian cancer, colon cancer (including colorectal cancer), lung cancer (including small cell lung cancer and non-small cell lung cancer), melanoma or pancreatic cancer.
